(19) Europäisches Patentamt
European Patent Office
Office européen des brevets

(11) EP 2 081 944 B1

(12) **FASCICULE DE BREVET EUROPEEN**

(45) Date de publication et mention de la délivrance du brevet:
**07.07.2010 Bulletin 2010/27**

(21) Numéro de dépôt: **07858552.8**

(22) Date de dépôt: **11.10.2007**

(51) Int Cl.:
*C07H 17/00* (2006.01) *A61K 31/7048* (2006.01)
*A61K 31/706* (2006.01)

(86) Numéro de dépôt international:
**PCT/FR2007/052122**

(87) Numéro de publication internationale:
**WO 2008/043962 (17.04.2008 Gazette 2008/16)**

# (54) NOUVEAUX DERIVES MORPHINIQUES

NEUE MORPHINDERIVATE

NOVEL MORPHINE DERIVATIVES

(84) Etats contractants désignés:
**AT BE BG CH CY CZ DE DK EE ES FI FR GB GR HU IE IS IT LI LT LU LV MC MT NL PL PT RO SE SI SK TR**

(30) Priorité: **12.10.2006 FR 0654247**

(43) Date de publication de la demande:
**29.07.2009 Bulletin 2009/31**

(73) Titulaire: **Neorphys**
**30900 Nîmes (FR)**

(72) Inventeurs:
- **LARBOURET, Karine**
  **34830 Clapiers (FR)**
- **LAHANA, Roger**
  **30900 Nîmes (FR)**
- **CASTEX, Cédric**
  **30900 Nîmes (FR)**

(74) Mandataire: **Gallois, Valérie**
**Cabinet BECKER & ASSOCIES**
**25, rue Louis Le Grand**
**75002 Paris (FR)**

(56) Documents cités:
**EP-A- 0 816 375** **WO-A-2005/063263**
**WO-A-2006/073396**

- **SALVATELLA, MARIONA ET AL: "A highly toxic morphine-3-glucuronide derivative" BIOORGANIC & MEDICINAL CHEMISTRY LETTERS ( 2004 ), 14(4), 905 -908 CODEN: BMCLE8; ISSN: 0960-894X, 2004, XP002434809 cité dans la demande**
- **HOUDI, ABDULGHANI A. ET AL: "3-O-acetylmorphine-6-O-sulfate: a potent, centrally acting morphine derivative" PHARMACOLOGY, BIOCHEMISTRY AND BEHAVIOR ( 1996 ), 53(3), 665 -71 CODEN: PBBHAU; ISSN: 0091-3057, 1996, XP002434810 cité dans la demande**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 2 081 944 B1

## Description

### DOMAINE DE L'INVENTION

La présente invention se rapporte à des nouveaux dérivés morphiniques, des compositions pharmaceutiques les contenant et à leur utilisation pour le traitement de la douleur et des dysfonctionnements sexuels.

### DESCRIPTION DU CONTEXTE DE L'INVENTION

La morphine est actuellement l'analgésique le plus utilisé dans le traitement des douleurs de moyenne et grande intensité. Cet opioïde est utilisé dans environ 80% des cas de douleur aiguë post-opératoire. Malgré une grande efficacité, l'utilisation de la morphine s'accompagne de nombreux effets secondaires indésirables, caractéristiques des opioïdes, tels la dépression respiratoire, les nausées, les vomissements, l'inhibition du transit intestinal, la dépendance et la tolérance (Minoru Nariata & coll., 2001, Pharmacol et Ther, 89 1-15).

On distingue trois classes principales de récepteurs aux opioïdes : Mu (μ), Kappa (κ) et Delta (δ) qui appartiennent à la famille des récepteurs couplés aux protéines G. Des analyses de Western Blot, FRET (Fluorescence Resonance Energy Transfer) et BRET (Bioluminescence Resonance Energy Transfert) ont mis en évidence des monomères et des dimères de ces trois types de récepteurs. (George SR & coll., J Biol Chem 2000, 275 : 26128-26135 ; Jordan BA & coll. , Nature 1999, 399 :697-700 ; Gomes I & coll., J Neurosci 2000, 20 : RC110.). Les dimères existent sous forme homodimère et hétérodimère. Cette oligomérisation intervient sur le rôle physiologique des récepteurs aux opioïdes et constitue une nouvelle cible thérapeutique à fort potentiel.

En effet, des dérivés opioïdes synthétisés sous forme dimère présentent une meilleure affinité et sont plus puissants que les formes monomères pour les récepteurs μ, δ et κ (Hazum & coll., Biochem Biophys Res Comm 1982, 104 :347-353). Leur activité analgésique après administration intraveineuse est plus importante et leur durée d'action plus longue (WO2005/063263). Cette dernière caractéristique permet de diminuer le nombre d'administrations, en conservant une activité analgésique maximale par rapport au monomère et autres opioïdes, tels que la morphine, qui ont une durée d'activité plus courte.

De plus, de nombreuses études (Gomes I & coll., J Neurosci 2000, 20 : RC110.) ont montré une colocalisation des récepteurs μ et δ, d'une part, et des récepteurs δ et κ, d'autre part, ce qui rend physiologiquement possible l'existence de formes hétérodimères de ces récepteurs. Cette hypothèse a été confirmée notamment par des analyses de FRET, d'immunoprécipitation et de profils de liaison (Rios & coll.,Pharm & Ther 2001, 92 : 71-87).

D'un point de vue pharmacologique, l'interaction d'une molécule avec les récepteurs aux opioïdes est responsable d'une activité analgésique plus ou moins importante et des effets secondaires.

Ainsi, la liaison aux récepteurs μ est en grande partie responsable de l'activité analgésique de la molécule. Il existe deux sous-types de récepteurs μ: le sous-type μ1 avec une forte affinité et une faible capacité et le sous-type μ2 avec une faible affinité et une forte capacité (Pasternak & Wood, 1986, Life Sci 38: 1889-1898). L'interaction avec les récepteurs μ1 se traduit par une réaction analgésique supraspinale et d'une diminution du turnover de l'acétylcholine, tandis que l'interaction avec les récepteurs μ2 provient d'une action analgésique spinale connue pour être responsable de la dépression respiratoire et de l'inhibition du transit intestinal.

Les récepteurs κ et δ interviennent en particulier dans la motilité intestinale et l'activité analgésique. De plus, leur inhibition contribue à diminuer le phénomène de dépendance et de dépression respiratoire (Rothman & coll., 2000, J Subst Abuse Treat 19 : 277-281, Shook & coll., 1990, Am Rev Respir Dis 142 : 895-909, J Neurosci 2005 Mar 23; 25 (12) : 3229-33. J Pharmacol Exp Ther. 2001 May; 297(2) : 597-605, J Pharmacol Exp Ther. 1998 Dec; 287(3) : 815-23).

Ces récepteurs sont présents dans le système nerveux central, en particulier au niveau de la corne dorsale de la moelle épinière mais également au niveau périphérique (Stein & coll., 1995, Ann Med, 27(2) : 219-21, Janso, Stein, Curr Opin Pharmacol, 2001, 1(1) : 62-65).

Les principaux effets secondaires observés, tels que la dépression respiratoire, les nausées, la dépendance et la tolérance, sont des effets centraux. Un moyen de limiter les effets indésirables tout en conservant une activité analgésique est de concevoir des opioïdes interagissant avec le système périphérique tout en diffusant progressivement dans le cerveau (C. Stein, 1990, J of Pain and Symptom Management 6(3) : 119-124; Zajaczowska R, Reg Anesth Pain Med, 2004, 29(5) : 424-429 ; Likar & coll., 1998, Pain 76(1-2) : 145-50 ; Tokuyama & coll., Life Sci, 1998, 62(17-18) : 1677-81 ; Junien, Aliment Pharmacol Ther, 1995, 9(2) : 117-26, Dehave-Hudkins & coll., J Pharmacol Exp The, 1999, 289(1) : 494-502, Stein & coll., N Engl J Med, 1991, 325 : 1123-1126). En effet, l'activation des récepteurs du système nerveux central semble rester la voie la plus efficace pour obtenir une analgésie comparable à celle obtenue avec la morphine.

Certains ligands bivalents ont été conçus et synthétisés pour interagir avec les hétérodimères μ/δ : ainsi, Daniels & al. ont étudié des ligands constitués d'un agoniste des récepteurs μ, l'oxymorphone, lié à un antagoniste des récepteurs δ, le naltrindole (Daniels & al., PNAS 2005, 102(52) : 19208, W0 2006/073396). Ce dernier est bien connu

dans la littérature comme antagoniste spécifique des récepteurs δ. Ils ont obtenu une activité analgésique du dimère comparable à l'oxymorphone par administration intracérébroventriculaire (icv) et une diminution significative des phénomènes de tolérance et de dépendance physique. De plus, il apparaît que ces dimères agissent au niveau central. En effet, leur ED50 et celle de la morphine sont comparables après administration par voies intraveineuse et icv.

On sait que la morphine subit un important métabolisme qui conduit à la formation notamment de morphine-6-glucuronide (M6G). Ce métabolite pénètre faiblement dans le cerveau en raison de son caractère hydrophile. Il présente une activité analgésique plus puissante que celle présentée par la morphine par administration centrale avec une diminution de la dépression respiratoire, des nausées et des vomissements (Paul & coll., 1989. J Pharmacol. Exp. Ther. 251 ; 477-483 ; Frances et al, 1992. J Pharmacol. Exp. Ther. 262 ; 25-31). Cependant, il peut induire, comme la morphine, le syndrome de dépendance. De plus, comme tous les métabolites, il est rapidement éliminé par l'organisme. La M6G possède une affinité μ plus forte (ou comparable suivant les études) que la morphine et une affinité κ plus faible (Current Topics in Medicinal Chemistry, 2005, 5, 585-594).

La M6G semble ainsi être un point de départ intéressant pour concevoir un opioïde aussi actif que la morphine sans les effets indésirables qu'elle entraîne.

La M6G a été produite par voie synthétique dès 1968 (Hidetoshi & coll., 1969. Biochem Pharm. 8(2) : 279-286) et ses propriétés analgésiques ont été mises en évidence dans de nombreux articles (par exemple Frances & coll., 1992. J Pharmacol. Exp. Ther. 262 ; 25-31). Les brevets EP 597 915 B1 et US 6046 313 décrivent la synthèse de la M6G et de dérivés de celle-ci notamment en position 3 et le brevet n° EP 1086114B1 porte sur un procédé de synthèse sélectif de l'anomère β de la M6G. La demande n° WO95/05831 décrit l'utilisation de M6G sous forme orale pour le traitement de la douleur.

Des dérivés de la M6G ont été décrits. Par exemple, le brevet n° EP 975 648 B1 décrit spécifiquement un dérivé de la M6G dans lequel la liaison entre les atomes de carbone 7-8 est saturée. La demande n° WO2005/063263 décrit des dérivés soufrés de la M6G, par substitution à l'aide d'un groupement porteur d'une fonction thiol ou d'un atome de soufre.

Des dérivés d'opioïdes conjugués à des sucres alternatifs à la M6G et ses dérivés ont été décrits dans la demande de brevet EP 816 375.

Cependant, il subsiste toujours la nécessité de développer des dérivés morphiniques présentant une activité analgésique plus puissante avec de moindres effets secondaires.

## DESCRIPTION DE L'INVENTION

La présente invention a pour principal objet de nouveaux dérivés de la M6G, de préférence sous forme de dimère ou de ligand bivalent, présentant une activité analgésique puissante, une durée d'action plus longue et moins d'effets secondaires que les opioïdes couramment utilisés. Ces dérivés ont été conçus :

- pour diffuser lentement au niveau central en jouant sur l'hydrophobicité des modifications chimiques apportées,
- pour interagir avec les récepteurs aux opioïdes présents sous forme homodimère et hétérodimère,
- pour présenter une forte affinité pour les récepteurs μ et inhiber les récepteurs κ et/ou δ en vue de diminuer certains effets secondaires.

Les inventeurs ont pu déterminer que l'ajout d'un groupement porteur d'une fonction thiol sur l'acide carboxylique du groupement glucuronide de la M6G et la modification de l'hydroxyle en position 3 par une fonction éther notamment permettaient d'obtenir des composés présentant une forte affinité μ et κ et une activité analgésique puissante. Ces propriétés sont en particulier valables avec les formes dimérisées ou les ligands bivalents qui sont obtenus par oxydation des fonctions thiol, formant ainsi un pont disulfure.

Il a été montré dans la littérature que les liaisons disulfures pouvaient améliorer les propriétés *in vivo* des molécules actives : ces liaisons, relativement stables dans le plasma, sont clivées dans les cellules (G. Saito & coll., Advanced Drug Delivery Reviews, 2003, 55, 199-215).

L'effet recherché par ces substitutions est une modification du profil pharmacologique par rapport aux différents dérivés connus de la M6G.

Le groupe en position 3 est choisi de façon à augmenter la lipophilie de la M6G et améliorer son activité analgésique centrale en favorisant son passage à travers la barrière hémato encéphalique (BHE). Cependant, il a été montré dans la littérature que certaines modifications de l'hydroxyle en position 3 sur le noyau morphinique provoquaient une perte d'activité analgésique, voire une toxicité (Abdulghani A Houdi et al.,1996, Pharm Biochem and Beh, 53 (3), 665-671 ; Salvatella et al, 2004, Biiorg & Med Chem Lett., 14, 905-908). Les inventeurs ont d'ailleurs montré qu'une substitution en position 3 par un groupe méthoxy (dérivé de la 6-codéïne-glucuronide) mène à un composé inactif et toxique (voir exemple 1). Le résultat obtenu pour les composés selon la présente invention est donc surprenant.

Une seconde application de ces dérivés de la M6G concerne les dysfonctions sexuelles et en particulier

l'éjaculation précoce chez l'homme. En effet, l'efficacité de l'administration d'analgésiques pour le traitement de l'éjaculation précoce a été montrée dans la littérature (Eledjam & coll., Cah Anesthesiol. 1991 ; 39(2) : 111-4, Safarinejad & coll. J Clin Psychopharmacol. 2006 ; 26(1) : 27-31). On peut citer l'application locale d'anesthésiques tels que la lidocaïne, l'injection de tramadol (Safarinejad & coll., US23186872A) ou l'utilisation de préparations pharmaceutiques contenant notamment un mélange Viagra/agoniste des récepteurs δ (US06974839).

La présente invention concerne donc un composé de formule (A) :

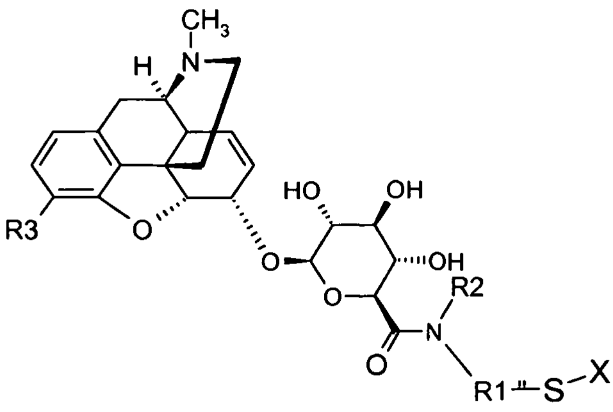
(A)

dans laquelle,

l'ensemble de l'entité (A), à l'exception du substituant X, est dénommé $M_{R3}$6G-NR1R2-S- ;

R1 représente un groupe alkyle en $C_1$-$C_{10}$, la chaîne alkyle étant éventuellement interrompue par un ou plusieurs hétéroatome(s) choisi(s) parmi O, S et N ;

R2 représente un hydrogène, un groupe alkyle en $C_1$-$C_5$, saturé ou insaturé, linéaire ou ramifié, ou un groupe aryle, hétéroaryle ou ($C_1$-$C_5$ )alkylaryle ;

R3 représente un groupe -O- éthyle : Y(C=Z)R ou YR, Y et Z représentant indépendamment l'oxygène ou le soufre, R étant un groupe alkyle en $C_1$-$C_6$ linéaire ou ramifié, saturé ou insaturé, à la condition que $R_3$ soit différent de O-$CH_3$;

X représente un hydrogène, un radical -S-R4-W, ou un radical $M_{R3}$6G-NR1R2-S-,

avec R4 étant un groupe alkyle en $C_1$-$C_8$ et pouvant comprendre des liaisons amide, ester, éther et,

W étant soit un antagoniste des récepteurs δ, soit un antagoniste des récepteurs κ,

ainsi que l'un de ses sels pharmaceutiquement acceptables.

Les inventeurs ont également décrit des composés tels que décrits ci-dessus dans lesquels R3 représente Y(C=Z)R ou_YR,_Y_et_Z représentant indépendamment l'oxygène ou le soufre, R étant un groupe alkyle en $C_1$-$C_6$ linéaire ou ramifié, saturé ou insaturé, à la condition que $R_3$ soit différent de O-$CH_3$.

Lorsque R1 est un alkyle substitué, le substituant peut être choisi parmi le groupe consistant en un groupe alkyle en $C_1$-$C_5$ saturé ou insaturé ; un groupe amino ; un groupe COOR5, un groupe CONR5R6, R5 et R6 représentant indépendamment l'hydrogène, un groupe alkyle en $C_1$-$C_{20}$, saturé ou insaturé, éventuellement substitué, un aryle, un hétéroaryle ; un alkyle en en $C_1$-$C_{20}$, de préférence en $C_1$-$C_{10}$, portant une fonction aldéhyde et/ou cétone.

Lorsque R2 est substitué, le substituant peut être un alkyle en $C_1$-$C_4$ saturé ou insaturé.

De préférence, lorsque W est un antagoniste des récepteurs δ, il est choisi parmi le groupe consistant en le naltrindole, le Naltriben, le 7-benzylidenenaltrexone (BNTX), et le 7-(5', 6'-benzo-2'-spiro-indanyl)naltrexone (BSINTX).

Lorsque W est un antagoniste des récepteurs κ, il est choisi parmi le groupe consistant en le 5'-guanidinonaltrindole et le nor-binaltorphimine (nor-BNI). Le radical W est lié à R4 de manière à conserver son activité antagoniste.

Lorsque X représente un radical $M_{R3}$6G-NR1R2-S-, les radicaux R1, R2, et R3 sur les deux radicaux $M_{R3}$6G-NR1R2-S- peuvent être identiques ou différents.

Un alkyle désigne un radical hydrocarboné saturé ou insaturé, linéaire ou ramifié, substitué ou non substitué. Par alkyle en $C_1$-$C_{10}$ est entendu un radical tel que défini ci-dessus présentant 1 à 10 atomes de carbone, de préférence un groupe sélectionné parmi un méthyle, éthyle, propyle, isopropyle, butyle, isobutyle, *tert*-butyle, pentyle, hexyle, heptyle, octyle, nonyle, et décyle. Par alkyle en $C_1$-$C_5$ est entendu un radical tel que défini ci-dessus présentant 1 à 5 atomes de carbone, par exemple un groupe sélectionné parmi un méthyle, éthyle, propyle, isopropyle, butyle, isobutyle, *tert*-butyle, et pentyle. Par alkyle en $C_1$-$C_4$ est entendu un radical tel que défini ci-dessus présentant 1 à 4 atomes de carbone, par exemple un groupe sélectionné parmi un méthyle, éthyle, propyle, isopropyle, butyle, isobutyle, et *tert*-butyle. Par alkyle en $C_1$-$C_6$ est entendu un radical tel que défini ci-dessus présentant 1 à 6 atomes de carbone, notamment un groupe sélectionné parmi un méthyle, éthyle, propyle, isopropyle, butyle, isobutyle, *tert*-butyle, pentyle, et hexyle. Par

alkyle en $C_2$-$C_6$ est entendu un radical tel que défini ci-dessus présentant 2 à 6 atomes de carbone, notamment un groupe sélectionné parmi un éthyle, propyle, isopropyle, butyle, isobutyle, *tert*-butyle, pentyle, et hexyle. Par alkyle en $C_1$-$C_8$ est entendu un radical tel que défini ci-dessus présentant 1 à 8 atomes de carbone, de préférence un groupe sélectionné parmi un méthyle, éthyle, propyle, isopropyle, butyle, isobutyle, *tert*-butyle, pentyle, hexyle, heptyle, et octyle. Par alkyle en $C_1$-$C_3$ est entendu un radical tel que défini ci-dessus présentant 1 à 3 atomes de carbone, de préférence un groupe sélectionné parmi un méthyle, éthyle, propyle, et isopropyle. Par alkyle en $C_2$-$C_3$ est entendu un radical tel que défmi ci-dessus présentant 2 à 3 atomes de carbone, de préférence un groupe sélectionné parmi un éthyle, propyle, et isopropyle. Par alkyle en $C_1$-$C_4$ est entendu un radical tel que défini ci-dessus présentant 1 à 4 atomes de carbone, de préférence un groupe sélectionné parmi un méthyle, éthyle, propyle, isopropyle, butyle, isobutyle, et *tert*-butyle.

Le terme « aryle » désigne un radical hydrocarboné aromatique, substitué ou non, ayant de préférence 6 à 14 atomes de carbone. De préférence, les radicaux aryles selon la présente invention sont choisis parmi le phényle, le naphtyle (par exemple 1-naphtyle ou 2-naphtyle), le biphényle (par exemple, 2-, 3- ou 4- biphényle), l'anthryle ou le fluorényle. Les groupes phényles, substitués ou non, sont tout particulièrement préférés.

Le terme « hétéroaryle » désigne un radical hydrocarboné aromatique comportant un ou plusieurs hétéroatomes tels que l'azote, le soufre et l'oxygène, substitué ou non, ayant de préférence 6 à 14 atomes de carbone. A titre d'exemple, on peut citer les groupements pyridinyle, pyridazinyle, pyrimidyle, pyrazyle, triazinyle, pyrrolyle, pyrazolyle, imidazolyle, (1,2,3)- et (1,2,4)-triazolyle, pyrazinyle, pyrimidinyle, tétrazolyle, furyle, thiényle, isoxazolyle, thiazolyle, isoxazolyle ou oxazolyle, etc.

Le terme « alkylaryle » désigne un radical du type aryle substitué par un groupement alkyle. Les termes « alkyle » et « aryle » répondent aux définitions précédemment énoncées. Des exemples de groupes alkylaryle sont notamment tolyle, mésythyle et xylyle.

Plus précisément, la présente invention concerne les dérivés de la morphine représentés par les formules (B), (C) et (D):

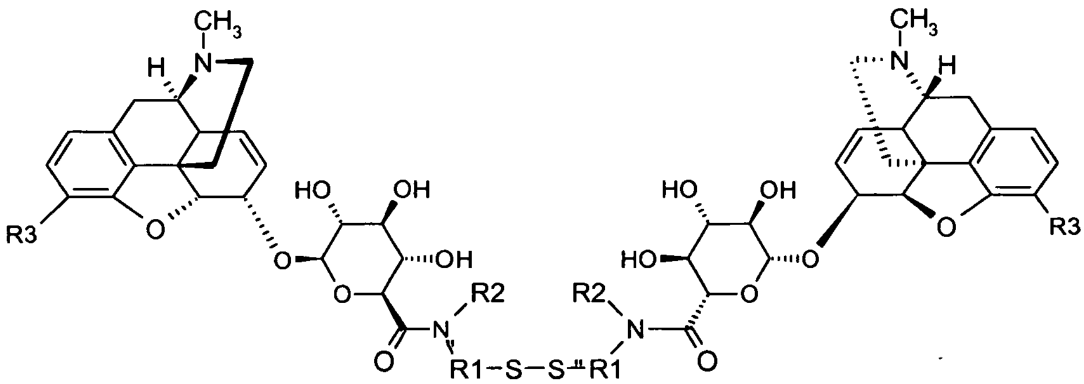

(B)

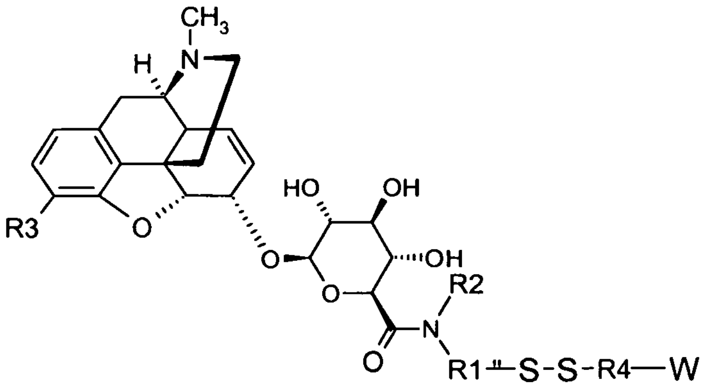

(C)

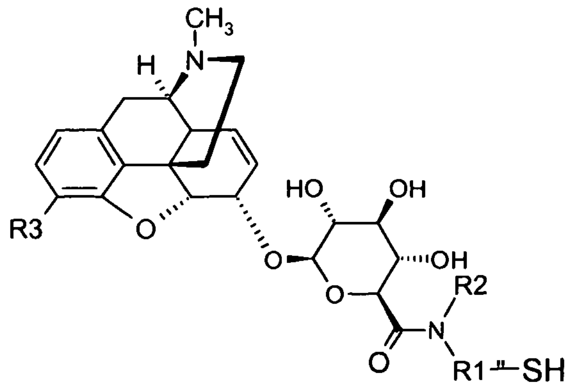

(D)

dans lesquelles R1, R2, R3, R4 et W sont comme définis dans la formule (A), les radicaux R1, R2, et R3 sur les deux radicaux $M_{R3}$6G-NR1R2-S- pouvant être identiques ou différents,
ainsi que l'un de leurs sels pharmaceutiquement acceptables.

Les inventeurs ont décrit des composés dans lesquels Y et Z dans le radical R3 sont des oxygènes. R dans le radical R3 peut être un alkyle en $C_2$-$C_6$ linéaire ou ramifié, saturé ou insaturé, de préférence un alkyle en $C_2$-$C_3$ linéaire ou ramifié, saturé ou insaturé, de préférence saturé. R3 peut être Y(C=Z)R avec R étant un groupe alkyle en $C_1$-$C_6$ linéaire ou ramifié, saturé ou insaturé, de préférence en $C_1$-$C_3$. En particulier, R3 peut être YR avec R étant un groupe alkyle en $C_2$-$C_6$ linéaire ou ramifié, saturé ou insaturé, de préférence en $C_2$-$C_3$.

Dans un mode de réalisation préféré, R4 représente un groupe $-(CH_2)_n$-C(O)-NH- ou $-(CH_2)_n$-NH- C(O)-, dans lequel n est un entier compris entre 1 et 8, de préférence entre 1 et 4.

Des composés préférés sont les composés de formules (A) à (D), dans lesquels les composés présentent une ou plusieurs des caractéristiques suivantes :

- le groupe R3 représente -OR, où en particulier R est un éthyle ou isopropyle ; et/ou
- R2 est un hydrogène ; et/ou
- R1 est un groupe alkyle linéaire $-(CH_2)_2$- ; et/ou
- R4 est un groupe $-(CH_2)_2$-C(O)-NH- ; et/ou
- W représente le naltrindo le.

Notamment, les inventeurs ont décrit des composés de formules (A) à (D), dans lesquels :

- R2 est un hydrogène et R1 est un groupe alkyle linéaire $-(CH_2)_2$- ;
  et/ou
- R2 est un hydrogène et R3 représente -OR, où en particulier R étant un éthyle ou isopropyle ; et/ou
- R4 est un groupe $-(CH2)_2$-C(O)-NH- et W représente le naltrindole ;
  et/ou
- R1 est un groupe alkyle linéaire $-(CH_2)_2$- et R3 représente -OR, où en particulier R étant un éthyle ou isopropyle ; et/ou
- R1 est un groupe alkyle linéaire $-(CH_2)_2$-, R2 est un hydrogène et R3 représente -OR, où en particulier R étant un éthyle ou isopropyle.

Dans un mode de réalisation particulier, le composé présente la formule (B) et les deux R2 sont un hydrogène et les deux R1 sont un groupe alkyle linéaire $-(CH_2)_2$-. Dans un autre mode de réalisation particulier, le composé présente la formule (C) et R2 est un hydrogène, R1 est un groupe alkyle linéaire $-(CH_2)_2$- et R4 est un groupe - $(CH2)_2$-C(O)-NH-.

Les carbones asymétriques présents dans les formules (A) à (D) peuvent être de configuration R ou S.

Les composés préférés sous forme de dimère sont représentés dans les structures (I), (II) ou (III).

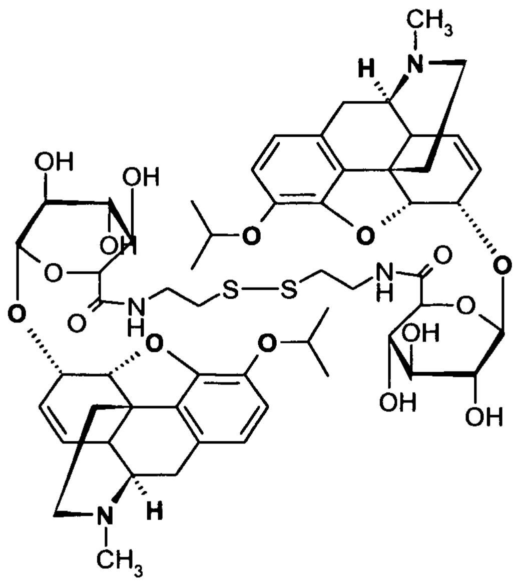

**(I) : M3iPro-6G-cya-cya-M3iPro-6G**

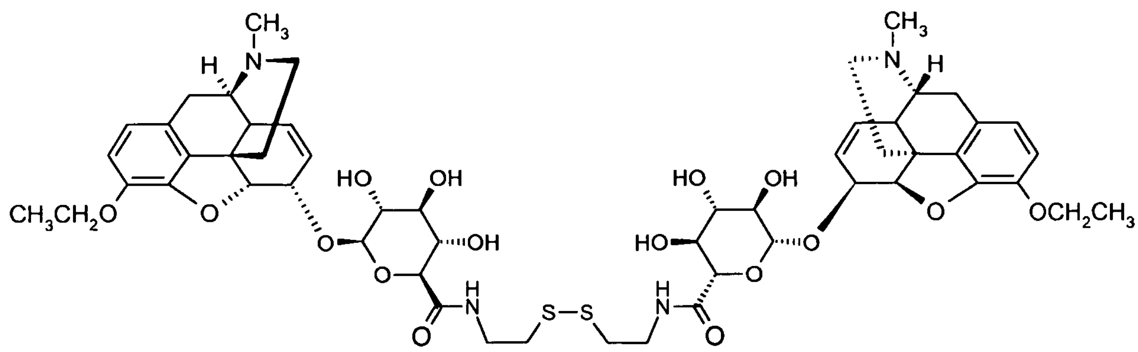

**(II) : M3Et-6G-cya-cya-M3Et-6G**

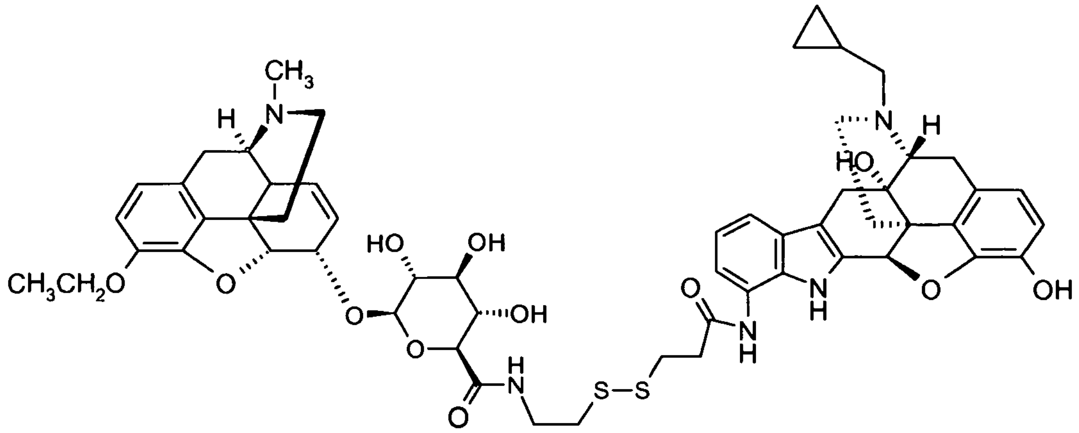

**(III) : M3Et-6G-cya-3MP-NTI.**

Le groupe éthyle peut être remplacé par un méthyle pour donner le composé C6G-cya-3MP-NTI.

Les composés de la présente invention peuvent être préparés par des méthodes bien connues de l'homme du métier.

L'invention concerne un composé selon la présente invention en tant que médicament.

L'invention concerne également une composition pharmaceutique contenant à titre de principe actif l'un des composés décrits ci-dessus ou l'un de ses sels pharmaceutiquement acceptables, tels que par exemple et de manière non limitative, un acétate, un sulfate ou un chlorhydrate.

La composition pharmaceutique selon l'invention se présentera sous une forme appropriée pour une administration :

- par voie parentérale, comme par exemple sous forme de préparations injectables par voie sous-cutanée, intraveineuse ou intramusculaire ;
- par voie orale, comme par exemple sous forme de comprimés, gélules, poudres, granulés, suspensions ou solutions orales, soit à libération immédiate soit à libération prolongée ou retardée ;
- par voie topique, en particulier transdermique, comme par exemple sous la forme de patch, de pommade ou de gel ;
- par voie intranasale, comme par exemple sous forme d'aérosols et sprays ;
- par voie rectale, comme par exemple sous forme de suppositoires ;
- par voir perlinguale ;
- par voie intraoculaire.

Le véhicule pharmaceutiquement acceptable peut être choisi parmi les véhicules utilisés de manière classique selon chacun des modes d'administration.

Dans un mode de réalisation, la composition peut comprendre en outre un autre principe actif.

Les inventeurs décrivent l'utilisation d'un composé de formule sélectionnée parmi les formules (A) à (D) et (I) à (III) ou d'un de ses sels pharmaceutiquement acceptables pour la fabrication d'un médicament utile pour le traitement de la douleur, en particulier pour le traitement de douleurs aiguës ou des douleurs chroniques, de douleurs neuropathiques, musculaires, osseuses, postopératoires, de la migraine, les douleurs du cancer, des lombalgies, des douleurs arthrosiques, des douleurs associées au diabète ou des douleurs associées au SIDA. L'invention concerne également l'utilisation d'un composé de formule sélectionnée parmi les formules (A) à (D) et (I) à (III) ou d'un de ses sels pharmaceutiquement acceptables pour la fabrication d'un médicament utile pour le traitement des dysfonctions sexuelles et en particulier l'éjaculation précoce.

Les inventeurs décrivent une méthode de traitement de la douleur chez un sujet comprenant l'administration d'une dose thérapeutiquement efficace d'un composé de formule sélectionnée parmi les formules (A) à (D) et (I) à (III) ou d'un de ses sels pharmaceutiquement acceptables. En particulier, la douleur est une douleur aiguë ou chronique, une douleur neuropathique, musculaire, osseuse, post-opératoire, une migraine, une douleur du cancer, une lombalgie, une douleur arthrosique, une douleur associées au diabète ou une douleur associée au SIDA. Par ailleurs, la présente invention concerne une méthode de traitement des dysfonctions sexuelles et en particulier l'éjaculation précoce chez un sujet comprenant l'administration d'une dose thérapeutiquement efficace d'un composé de formule sélectionnée

parmi les formules (A) à (D) et (I) à (III) ou d'un de ses sels pharmaceutiquement acceptables.

Les inventeurs décrivent enfin un composé de formule sélectionnée parmi les formules (A) à (D) et (I) à (III) ou d'un de ses sels pharmaceutiquement acceptables pour le traitement de la douleur ou le traitement des dysfonctions sexuelles.

## DESCRIPTION DES FIGURES

Figure 1 : Schéma de synthèse des C6G-Cya, M3Et-6G-Cya, de M3Et-6G-Cya-Cya-M3Et-6G, M3iPro-6G-cya-cya-M3iPro-6G et M3Et-6G-cya-3MP-NTI.
Figure 2 : Activité analgésique des composés dans le test du Tail Flick.
Figure 3 : Activité analgésique des composés dans le test de la Hot Plate.
Figure 4 : Influence des composés sur le transit intestinal

L'invention est illustrée de manière non-limitative par les exemples ci-dessous.

## EXEMPLES

### A. Synthèse

Les différents procédés de synthèse mis en oeuvre pour obtenir les produits considérés sont détaillés ci-après. Les schémas réactionnels sont représentés sur la figure 1.

Les réactions ont été suivies par chromatographie liquide haute pression (HPLC) analytique en phase inverse et spectrométrie de masse MALDI-TOF. Les produits ont été caractérisés et leur pureté déterminée par HPLC analytique en phase inverse et spectrométrie de masse.

### Exemple 1 : Synthèse de C6G-cya (exemple de référence)

Dans un réacteur, 2 équivalents molaires de cystamine ont été dissous dans du diméthylformamide (DMF) anhydre à 138g/l. 1 équivalent molaire de codéine-6-glucuronide.TFA commerciale a été additionné après dissolution dans du DMF anhydre à 47g/l.

4 équivalents molaires de diisopropyléthylamine (DIEA) ont été ajoutés et le réacteur a été placé dans un bain d'eau glacée (0°C). 1,2 équivalents molaires de benzotriazole-1-yl-oxy-tris-pyrrolidinophosphonium hexafluorophosphate (PyBOP) préalablement solubilisés dans du DMF à 225g/l ont été introduits goutte à goutte au milieu réactionnel qui est ensuite agité à température ambiante pendant 3h.

Le pont disulfure a alors été réduit en ajoutant 2,5 équivalents molaires de tris(2-carboxyéthyl)phosphine (TCEP) préalablement solubilisés dans de l'eau/TFA0,1% à 214g/l. Après 2 heures de réaction, le produit a été purifié par HPLC préparative.

10,3 mg de C6G-cya ont été obtenus après lyophilisation : $[M+H]^+$= 535 - $M_{TFA}$= 648 - Pureté : 98% - Rdt=93%.

### Exemple 2 :Synthèse de M3Et-6G-cya

#### Synthèse M3ET-6G :

Dans un réacteur, 1 équivalent molaire de morphine-6-glucuronide.$2H_2O$ (M6G) a été introduit et solubilisé dans de l'eau à 100g/l. 5 équivalents de carbonate de césium ont été ajoutés et le milieu a été agité 5 minutes à température ambiante.

Un volume de dichlorométhane identique à celui d'eau a été introduit dans le mélange. 5 équivalents de bromoéthane et 2 équivalents de tetrabutylammonium hydrogen sulfate (TBAHS) ont été additionnés successivement. Le milieu a été agité à température ambiante 72 heures. Le produit a alors été purifié par HPLC préparative.

Une expérience Nuclear Overhauser Effect (NOE) par Résonance Magnétique Nucléaire (RMN) du proton a montré que le groupement éthyle était porté par l'atome d'oxygène en position 3 du dérivé morphinique. En effet, l'irradiation contrôlée du groupement éthyle a entraîné une perturbation des signaux des protons aromatiques du phénol.

45,8 mg de M3Et-6G ont été obtenus : $[M+H]^+$= 490 - $M_{TFA}$= 603 - Pureté : 93% - Rdt=55%.

**Synthèse M3Et-6G-cya**

Dans un réacteur, 2 équivalents molaires de cystamine.dihydrochloride ont été solubilisés dans du DMF à 71g/l. 1 équivalent de M3Et6G préalablement solubilisé dans du DMF à 96g/l a été ajouté. Le milieu a été dilué avec du DMF et 4 équivalents molaires de DIEA ont été ajoutés. Le milieu réactionnel a été refroidi à 0°C et 1,2 équivalents molaires de PyBOP préalablement solubilisés dans du DMF à 23g/l ont été introduits goutte à goutte.

Après 3 heures d'agitation à température ambiante, 2,5 équivalents molaires de TCEP solubilisés dans de l'eau/TFA 0,1% à 21g/l ont été ajoutés. Après 4 heures d'agitation, la réduction est terminée. Le produit a été purifié par HPLC préparative.

46,9 mg de M3Et-6G-cya ont été obtenus : $[M+H]^+$= 549 - $M_{TFA}$= 662 - Pureté : 95% - Rdt=89%.

**Exemple 3 : Synthèse de M3Et-6G-cya-cya-M3Et-6G**

Dans un réacteur, 1 équivalent molaire de cystamine.dihydrochloride a été solubilisé dans du DMF à 86g/l. 2 équivalents molaires de M3Et-6G-cya préalablement solubilisés dans du DMF à 97 g/l ont été ajoutés. 5 équivalents molaires de DIEA pure ont alors été introduits et le milieu a été refroidi à 0°C dans un bain de glace. 2,4 équivalents molaires de PyBOP solubilisés dans du DMF à 22,9 g/l ont été additionnés goutte à goutte. Le milieu a été agité 3 heures à température ambiante. Le produit a été purifié par HPLC préparative.

40,5 mg de dimère M3Et-6G-cya-cya-M3Et-6G ont été obtenus : $[M+H]^+$= 1096 - $M_{TFA}$= 1322 - Pureté : 95% - Rdt=81%.

**Exemple 4 : Synthèse de M3iPro-6G-cya-cya-M3iPro-6G (exemple de référence)**

**Synthèse M3iPro-6G :**

Dans un ballon de 10ml, 1 équivalent molaire de M6G est dissous dans de l'eau à 100g/l. 5 équivalents de carbonate de cesium, 2 équivalents de TBAHS, 1 ml de dichlorométhane et 5 équivalents de bromoisopropane sont ajoutés. Le milieu est agité à 45°C, reflux du dichlorométhane pendant une nuit.
Le dichlorométhane est évaporé et le produit attendu est purifié par HPLC préparative.

61,7 mg de M3ipro6G ont été obtenus : $[M+H]^+$= 504 - $M_{TFA}$= 617 - Pureté : 95% - Rdt=63%.

**Synthèse M3iPro-6G-cya-cya-M3iPro-6G**

Dans un tube falcon, 1 équivalent de cystamine 2HCl est solubilisé dans du DMF à 11,2g/l. 2 équivalents de M3iPro-6G préalablement dissous dans du DMF à 100g/l sont ajoutés ainsi que 5 équivalents de DIEA pure. Le milieu réactionnel est refroidi à 0°C et agité 5 minutes. Puis 2,4 équivalents de PyBOP préalablement dissous dans du DMF à 240g/l sont ajoutés et le milieu est agité à température ambiante pendant 1 h.

Le dimère est purifié par HPLC préparative.

46,4 mg de M3iPro-6G-cya-cya-M3iPro-6G sont obtenus: $[M+H]^+$= 1123 - $M_{TFA}$= 1350 - Pureté : 95% - Rdt=85%.

**Exemple 5 : Synthèse de M3Et-6G-cya-3MP-NTI**

**Synthèse 7'-Nitronaltrindole**

Dans un ballon sont introduits 1 équivalent de Naltrexone HCl $2H_2O$ et 1 équivalent de (2-nitrophenyl)-hydrazine dans un mélange 50/50 d'acide chlorhydrique 37% et d'acide acétique glacial à 57g/l. Le mélange est agité 6h30 à 85°C puis refroidi à 0°C, neutralisé par une solution de $NaHCO_3$ saturée et extrait par 3 fois à l'acétate d'éthyle. Les phases organiques sont réunies, séchées sur sulfate de sodium, filtrées et évaporées sous pression réduite. Le solide obtenu est alors purifié par HPLC préparative.

90mg de 7'-nitronaltrindole sont obtenus : $[M+H]^+$= 460 - $M_{TFA}$= 573 - Pureté : 92% - Rdt=43%.

**Synthèse du 7'-aminonaltrindole**

Dans un falcon de 50ml est introduit 1 équivalent de 7'-nitronaltrindole dissous dans de l'éthanol à 34,6g/l. Un excès de Nickel de Raney (50% en suspension dans l'eau) et 10 équivalents d'hydrazine hydratée sont ajoutés goutte à goutte. Après 1h de réaction, le milieu est centrifugé, la phase alcool est récupérée et l'éthanol est évaporé sous pression réduite. Le résidu obtenu est repris dans un minimum d'eau (TFA 0,1%) / acétonitrile (TFA 0,1%) 50/50 et

injecté en HPLC préparative.

44,1 mg de produit sont obtenus : $[M+H]^+$= 430 - $M_{TFA}$= 657 - Pureté : 95% - Rdt=43%.

**Synthèse Fmoc-cystéamine-3MP**

Dans un ballon sont mis en solution 2 équivalents de Fmoc-OSu dans de l'acétone à 320g/l. 1 équivalent de cystamine 2HCl et 1,6 équivalents de carbonate de sodium sont ajoutés avec un volume d'eau identique à celui d'acétone. Le mélange prend en masse. Un mélange eau/acétone 50 /50 est ajouté pour doubler le volume de solvant.

Après 2h30 d'agitation à température ambiante, l'acétone est évaporée au rotavapor et la suspension obtenue est redissoute dans du dichlorométhane. La phase aqueuse est extraite au dichlorométhane et les phases organiques lavées successivement par du $KHSO_3$ et du NaCl saturé.

La phase organique finale est alors séchée sur sulfate de sodium, filtrée et évaporée sous pression réduite pour donner 2,53g de Fmoc-cystamine : $[M+H]^+$= 598 - Pureté : 95% - Rdt=96%.

La réduction du pont disulfure est réalisée en dissolvant 1 équivalent de Fmoc-cystamine dans du DMF à 25g/l et en ajoutant 2,5 équivalents de Tris(2-carboxyéthyl)phosphine (TCEP) préalablement dissous dans de l'eau (0,1%TFA) à 400g/l. Après 1h d'agitation à température ambiante, un large excès d'eau est ajouté et le milieu est extrait au dichlorométhane. Les phases organiques sont réunies, lavées au NaCl saturé, séchées sur $Na_2SO_4$ et filtrées sur fritté. Le dichlorométhane est évaporé. Le produit est alors précipité en ajoutant de l'eau (0,1%TFA), filtré sur fritté et dissous dans un mélange $H_2O$ (0, 1 %TFA)/ACN (0, 1 %TFA) (25/75) puis lyophilisé.

252 mg de produit sont obtenus : $[M+H]^+$= 300 - Pureté : 95% - Rdt=50%.

3 équivalents de 2-2'-dithiodipyridine dissous dans du dichlorométhane à 122g/l sont placés à 0°C.

1 équivalent de Fmoc-cystéamine préalablement dissous dans du dichlorométhane à 18g/l est ajouté goutte à goutte pendant 10 minutes.

Après 3h de réaction à température ambiante, le milieu est de nouveau placé à 0°C et 5,5 équivalents d'acide 3-mercaptopropionique dilués dans du dichlorométhane à 18µl/ml sont additionnés goutte à goutte pendant 20 minutes. Après une nuit de réaction à température ambiante, le dichlorométhane est évaporé sous pression réduite. Le résidu obtenu est purifié par HPLC préparative.

231 mg de Fmoc-cystéamine-3MP sont obtenus : $[M+H]^+$= 400 - Pureté : 95% - Rdt=68%.

**Synthèse de la cystéamine-3MP-7'aminonaltrindole :**

1 équivalent de 7' aminonaltrindole est mis en solution dans du DMF à 70g/l. 2,1 équivalents de Fmoc-cystéamine-3MP dissous dans du DMF à 66g/l et 6 équivalents de DIEA pure sont ajoutés. Le milieu est placé à 0°C sous agitation. 1,1 équivalents de PyBOP préalablement dissous dans du DMF à 200g/l sont additionnés goutte à goutte. Après 60 minutes de réaction, le milieu est purifié par HPLC préparative. 22,5 mg de Fmoc-cystéamine-3MP-7'aminonaltrindole sont obtenus : $[M+H]^+$= 815 - $M_{TFA}$= 928 - Pureté : 94% - Rdt=36%.

1 équivalent de Fmoc-cystéamine-3MP-7'aminonaltrindole est mis en solution dans du DMF à 15g/l. 20 équivalents de DEA pure sont ajoutés et le milieu est agité pendant 30 minutes à température ambiante.

Le milieu est purifié par HPLC préparative.

9,3 mg de cystéamine-3MP-7'aminonaltrindole sont obtenus : $[M+H]^+$= 593 - $M_{TFA}$= 820 - Pureté : 81% - Rdt=43%.

**Synthèse de M3Et-6G-cya-3MP-NTI**

1 équivalent de Cya-3MP-7'aminonaltrindole est dissous dans du DMF à 70g/l. 1 équivalent de M3Et-6G dissous dans du DMF à 70g/l est ajouté ainsi que 5 équivalents de DIEA. Le milieu est placé à 0°C et 1,2 équivalents de PyBOP dissous dans du DMF à 200g/l sont ajoutés goutte à goutte sur le mélange. Après 45 minutes sous agitation à température ambiante, le milieu est purifié par HPLC préparative.

11 mg de chimère sont obtenus : $[M+H]^+$= 1064 - $M_{TFA}$= 1291 - Pureté : 95% - Rdt=67%.

**B. Etude de l'affinité aux récepteurs opioïdes**

**B.1/ Mode Opératoire**

L'affinité de la M6G et des dérivés C6G-cya, M3Et-6G-cya et M3Et-6G-cya-cya-M3Et-6G a été comparée pour les trois sous-types de récepteurs aux opioïdes µ, δ et K.

Pour déterminer l'affinité aux récepteurs µ, des homogénats de membrane cellulaire (récepteurs µ humains transfectés sur des cellules HEK-293) ont été incubés à 22°C pendant 120 minutes avec 0,5 nM de $[^3H]$[D-Ala, N-MePhe,

Gly(ol)]enképhaline (DAMGO) en l'absence ou en présence d'un des composés selon l'invention dans un tampon contenant 50 mM Tris-HCl (pH 7,4) et 5mM $MgCl_2$.

Pour déterminer l'affinité aux récepteurs κ, des homogénats de membrane de cervelets de cobaye (250 μg de protéine) ont été incubés à 22°C pendant 80 minutes avec 0,7 nM [$^3$H]U-69593 en l'absence ou en présence d'un des composés selon l'invention dans un tampon contenant 50 mM Tris-HCl (pH 7,4), 10mM $MgCl_2$ et 1 mM d'EDTA.

Pour déterminer l'affinité aux récepteurs δ, des homogénats de membrane cellulaire (récepteurs δ humains transfectés sur des cellules CHO) ont été incubés à 22°C pendant 120 minutes avec 0,5 nM de [$^3$H]DADLE en l'absence ou en présence d'un des composés selon l'invention dans un tampon contenant 50 mM Tris-HCl (pH 7,4) et 5mM $MgCl_2$.

La liaison non spécifique a été déterminée en présence de 10 μM de naltrexone. Après incubation, les échantillons ont été filtrés rapidement sur des fibres de verre (GF/B, Packard) préalablement incubées avec 0,3% de polyéthylèneimine et rincées plusieurs fois avec 50 mM de Tris-HCl froid en utilisant un « 96-sample cell harvester » (Unifilter, Packard). Les filtres ont été ensuite séchés et la radioactivité a été comptée.

L'activité agoniste/antagoniste sur les récepteurs κ des composés selon l'invention a été évaluée en utilisant des segments prostatiques du canal déférent de lapin, étirés et stimulés par un courant électrique de 0,1 Hz pendant 1msec.

Pour tester une activité agoniste, une réponse contrôle (pic de contractions du tissu) a été obtenue en exposant le tissu à une forte concentration (0,1 μM) en U-69593, un agoniste spécifique des récepteurs κ.

Les tissus ont ensuite été exposés à des concentrations croissantes du composé selon l'invention ou de l'agoniste spécifique. Les différentes concentrations ont été cumulées et chacune est restée au contact du tissu jusqu'à obtenir une réponse stable ou 15 minutes au maximum.

Si une réponse caractéristique de l'agoniste était observée (c'est-à-dire une inhibition des contractions), un antagoniste de référence, la nor-binaltorphimine (nor-BNI, 0,001 μM), était testée à la plus haute concentration du composé selon l'invention pour confirmer l'implication des récepteurs κ dans la réponse.

Pour tester une activité antagoniste, une réponse contrôle (pic de contractions du tissu) a été obtenue en exposant le tissu à une forte concentration (0,1 μM) en agoniste spécifique U-69593.

Les tissus ont été ensuite exposés à des concentrations croissantes du composé selon l'invention ou de l'antagoniste spécifique nor-BNI. Les différentes concentrations ont été cumulées et chacune est restée au contact du tissu jusqu'à obtenir une réponse stable ou 15 minutes au maximum.

Une réponse antagoniste a été observée si l'amplitude des pics de contractions était semblable à celle observée avec la nor-BNI.

Le paramètre mesuré était un changement maximal d'amplitude de pics de contractions provoquées par une stimulation électrique à différentes concentrations en composé.

**B.2/ Résultats**

Les résultats d'affinité aux récepteurs μ, δ et κ sont rapportés dans le tableau 1 ci-dessous :

**Tableau 1 : Affinité aux récepteurs μ, δ et κ :**

| Composés | Ki (nM) Récepteur μ | Ki (nM) Récepteur δ | Ki (nM) Récepteur K |
|---|---|---|---|
| Morphine | 12 | 150 | 150 |
| C6G-cya | 11 | NC | 100 |
| M3Et-6G-cya | 13 | NC | 220 |
| M3Et-6G-cya-cya-M3Et-6G | 14 | >1000 | 25 |
| NC : Non mesuré car moins de 25% d'inhibition aux plus fortes concentrations. | | | |

Les résultats montrent :

- une affinité des composés selon l'invention pour les récepteurs μ comparable à celle de la morphine.
- une perte d'affinité des composés selon l'invention pour les récepteurs δ par rapport à la morphine.
- une affinité des composés monomères C6G-cya et M3Et-6G-cya pour les récepteurs κ comparable à celle de la morphine et une amélioration de l'affinité (facteur 6) pour le dimère M3Et-6G-cya-cya-M3Et-6G.

L'activité agoniste/antagoniste du composé M3Et-6G-cya-cya-M3Et-6G a été mesurée comme décrit ci-dessus ( § B1): le composé selon l'invention se comporte comme un antagoniste des récepteurs κ.

Les résultats d'activité agoniste/antagoniste sont présentés dans le tableau 2 ci-dessous :

**Tableau 2 : Activité agoniste/antagoniste sur le récepteur κ : Evaluation de l'activité agoniste :**

| Composés | Réponse Control à U-69593 (0, 1μM) | Réponse à une concentration croissante de composé | | | | + nor-BNI (1nM) |
|---|---|---|---|---|---|---|
| M3Et-6G-cya-cya-M3Et-6G | 100 | 0,03 μM | 0,3 μM | 3 μM | 30μM | 10 μM |
| | | 0 | 0 | -4 | -3 | NC |
| U-69593 | 100 | 0,01 μM | 0,03μM | 0,1μM | | 0,1 μM |
| | | 18 | 46 | 100 | | -6 |

**Evaluation de l'activité antagoniste :**

| Composés | Réponse Control à U-69593 (0,1μM) | Réponse à une concentration croissante de composé | | | |
|---|---|---|---|---|---|
| M3Et-6G-cya-cya-M3Et-6G | 100 | 0,03 μM | 0,3 μM | 3 μM | 30 μM |
| | | 100 | 100 | 92 | 71 |
| nor-BNI | 100 | 0,1 nM | 0,3nM | 1nM | |
| | | 80 | 55 | -1 | |
| Les réponses sont exprimées en % de la réponse contrôle en U-69593 (diminution de l'amplitude des contractions) | | | | | |

## C. Etude de l'activité analgésique

### C.1/ Mode opératoire

L'activité analgésique a été déterminée par le test du « Tail Flick » et le test de la « Hot Plate ».

Le test du « Tail flick » (test de D'Amour et Smith, 1941, Pharmacol Exp Ther ; 72 : 74-79) consiste à placer la queue d'une souris après administration d'un produit au point focal d'une source infrarouge de façon à produire un stimulus nociceptif (température de surface de 55°C). Le temps de réaction (TR) de la souris (latence entre le moment où le faisceau lumineux est déclenché et le moment où la souris retire sa queue) a été mesuré en duplicate à huit temps différents allant de 15 minutes à 480 minutes après l'administration du produit. Un temps maximum de 10 secondes a été choisi comme temps maximum de réaction de manière à ne pas induire de dommage tissulaire chez les animaux.

Les produits ont été administrés par voie intraveineuse à des doses comprises entre 0,5 et 8 mg/kg (8 souris par groupe).

Deux mesures de temps de réaction ont été réalisées avant administration du produit pour chaque souris et permettent d'établir un temps de base.

Le test du « Hot Plate » (test de la plaque chauffante) consiste à placer une souris sur une plaque dont la température a été préréglée à 54°C et à mesurer le temps d'apparition d'un des comportements suivants :

- saut faisant intervenir au moins 1 des 4 membres
- léchage des pattes antérieures ou postérieures
- dépassement de 30 secondes sur la plaque chauffante sans réaction de la part de l'animal
- activation de la vitesse de déplacement.

Le temps de réaction a été mesuré à huit temps différents allant de 15 minutes à 480 minutes après l'administration du produit.

Un temps de base a été déterminé pour chaque souris avant l'administration du produit étudié.

Les produits sont administrés par voie intraveineuse à des doses comprises entre 0,5 et 8 mg/kg (8 souris par groupe).

**C.2/ Résultats**

Concernant le test du « Tail Flick », les résultats obtenus pour la morphine, M3Et-6G-cya, M3Et-6G-cya-cya-M3Et-6G, C6G-cya-cya-C6G, M3iPro-6G-cya-cya-M3iPro-6G et M3Et-6G-cya-3MP-NTI exprimés en moyenne par groupe du % MPE ± S.E.M, sont représentés dans la figure 2.

Le % MPE représente le pourcentage d'effet maximal possible et correspond à la formule suivante :

$$\%MPE = \frac{(TR_{post\text{-}administration} - TR_{pré\text{-}administration})}{(TR_{max} - TR_{ré\text{-}administration})} \times 100$$

Pour chacun des produits, l'ED50 (dose active chez 50% des animaux) et l'AUC (aire sous la courbe) ont été calculées et sont représentées ci-dessous dans les tableaux 3 (ED50) et 4 (AUC).

**Tableau 3 : ED50 calculée à partir du test du Tail Flick :**

| ED50 mg/Kg | | |
|---|---|---|
| Composé | Effet max | ED50 |
| Morphine | 30 min | 3,273 |
| M(3Et)6G-Cya | 120 min | 2,042 |
| M3Et-6G-cya-cya-M3Et-6G | 120 min | 1,318 |
| C6G-cya-cya-C6G | 30 min | ND |
| M3iPro-6G-cya-cya-M3iPro-6G | 60 min | 1,7 |
| M3Et-6G-cya-3MP-NTI | 120 min | 2,9 |
| ND : non déterminé (absence de réponse analgésique) | | |

**Tableau 4 : AUC calculée à partir du test du Tail Flick :**

| AUC | | | | | |
|---|---|---|---|---|---|
| **Dose (mg/kg)** | **-** | **-** | **1,7** | **3,4** | **6,8** |
| Morphine | - | - | 60,62 | 589,54 | 1529,18 |
| **Dose (mg/kg)** | **0,5** | **1** | **2** | **4** | **8** |
| M(3Et)6G-Cya | - | 1047,85 | 2538,93 | 5012,90 | 6059,22 |
| M3Et-6G-cya-cya-M3Et-6G | 617,8 | 1233,75 | 4824,51 | 5915,16 | 5817,46 |
| C6G-cya-cya-C6G | 440 | 147 | 500 | 281 | 251 |
| M3iPro-6G-cya-cya-M3iPro-6G | - | 1223 | 4242 | 5685 | - |
| M3Et-6G-cya-3MP-NTI | - | - | 2576 | 4410 | 5733 |

Concernant le test du « Hot Plate », les résultats obtenus pour la morphine, M3Et-6G-cya et M3Et-6G-cya-cya-M3Et-6G, exprimés en moyenne par groupe du % MPE ± S.E.M, sont représentés dans la figure 3.

Pour chacun des produits, l'ED50 (dose active chez 50% des animaux) et l'AUC (aire sous la courbe) ont été calculées et sont représentées ci-dessous dans les tableaux 5 (ED50) et 6 (AUC).

**Tableau 5 : ED50 calculée à partir du test de la Hot Plate :**

| ED50 mg/Kg | | |
|---|---|---|
| Composé | Effet max | ED50 |
| Morphine | 30 min | 3,802 |
| M(3Et)6G-Cya | 120 min | 2,754 |
| M3Et-6G-cya-cya-M3Et-6G | 120 min | 1,349 |

**Tableau 6 : AUC calculée à partir du test de la Hot Plate :**

| AUC | | | | | |
|---|---|---|---|---|---|
| Dose (mg/Kg) | - | - | 1,7 | 3,4 | 6,8 |
| Morphine | - | - | 931,55 | 986,99 | 4613,09 |
| Dose (mg/kg) | 0,5 | 1 | 2 | 4 | 8 |
| M(3Et)6G-Cya | - | 1912,70 | 4962,00 | 9618,55 | 16359,84 |
| M3Et-6G-cya-cya-M3Et-6G | 1182,74 | 1437,18 | 4928,30 | - | - |

Les résultats montrent que les composés selon l'invention ont une activité analgésique au moins supérieure à celle de la morphine. En effet, leur ED50 est comprise entre 1,3 et 2 mg/kg à comparer à 3,3 mg/kg pour la morphine.

De plus, on observe une durée d'activité analgésique nettement plus longue pour les composés M3Et-6G-cya et M3Et-6G-cya-cya-M3Et-6G que pour la morphine. En effet, les composés selon l'invention sont actifs au moins deux fois plus longtemps que la morphine.

**D. Etude du transit intestinal chez la souris**

**D.1/ Mode opératoire**

Des souris éveillées sont gavées par introduction dans l'oesophage d'une sonde de gavage montée sur une seringue de 1ml. 600µl de pâte de gavage (constituée notamment de charbon actif) sont introduits lentement. 30 min après le gavage, les animaux sont sacrifiés. Après incision de la paroi abdominale, les intestins sont exposés sur toute leur longueur. La distance entre le cardia et le rectum est mesurée (longueur totale) ainsi que la distance entre le cardia et le front du marqueur.

L'effet du composé M3Et-6G-cya-cya-M3Et-6G sur le phénomène de constipation a été mesuré à l'effet max après injection iv à l'ED50 puis comparé à celui provoqué par la morphine dans les mêmes conditions.

Afin de se rapprocher d'un cas clinique de douleur post-opératoire où le patient a besoin d'un effet analgésique de plusieurs heures, le protocole suivant a été utilisé :

Le composé M3Et-6G-cya-cya-M3Et-6G et la morphine sont injectés à deux fois l'ED50.

Afin de maintenir une analgésie maximale pendant 6h30, une dose de morphine est injectée toutes les heures, ce qui peut correspondre à une pompe à morphine en milieu hospitalier. Il n'est pas nécessaire d'injecter de dose supplémentaire de M3Et-6G-cya-cya-M3Et-6G puisque l'activité est maximale pendant au moins 6 heures.

Le transit est alors mesuré 30 min après le gavage des souris à 6 heures après administration.

Une seconde mesure est réalisée 2h30 après la dernière injection de morphine, soit 8h30 après l'injection de M3Et-6G-cya-cya-M3Et-6G. Enfin des mesures sont réalisées à 10h et 12h après l'injection de M3Et-6G-cya-cya-M3Et-6G afin d'évaluer la vitesse de retour au transit normal.

**D.2/ Résultats**

Le pourcentage de transit est calculé comme :

la distance du front du marqueur x 100 / longueur totale.

Les données sont présentées dans la figure 4.

Aucune différence significative sur le transit n'est observée entre le composé M3Et-6G-cya-cya-M3Et-6G et la morphine à l'effet analgésique maximal après une injection à l'ED50 (inhibition du transit de l'ordre de 90%).

Dans le cas où l'analgésie est maintenue maximale pendant 6h30, l'inhibition du transit est nettement inférieure

avec le composé M3Et-6G-cya-cya-M3Et-6G (52 à 56%) par rapport à la morphine (82%)

Lorsque l'activité analgésique est maintenue maximale avec la morphine, le transit est complètement arrêté. Il ne se remet en route que lorsque les injections de morphine sont arrêtées, ce qui se traduit également par une chute de l'activité analgésique.

Dans le cas du composé M3Et-6G-cya-cya-M3Et-6G, le transit est seulement ralenti alors que l'activité analgésique reste maximale. La vitesse de transit redevient normale entre 10 et 12 heures avec une analgésie de 50%.

## Revendications

1. Composé de formule (A) :

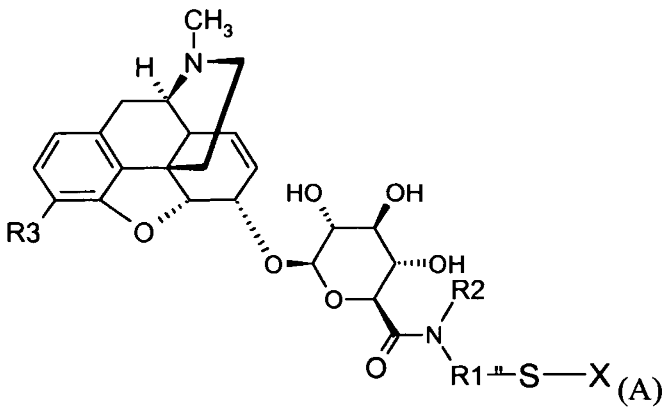

(A)

dans laquelle,
l'ensemble de l'entité (A), à l'exception du substituant X, est dénommé $M_{R3}$6G-NR1R2-S- ;
R1 représente un groupe alkyle en $C_1$-$C_{10}$ saturé ou insaturé, linéaire ou ramifié, la chaîne alkyle étant éventuellement interrompue par un ou plusieurs hétéroatome(s) choisi(s) parmi O, S et N ;
R2 représente un hydrogène, un groupe alkyle en $C_1$-$C_5$, saturé ou insaturé, linéaire ou ramifié, ou un groupe aryle, hétéroaryle ou ($C_1$-$C_5$)alkylaryle ;
R3 représente un groupe -O-éthyle;
X représente un hydrogène, un radical -S-R4-W, ou un radical $M_{R3}$6G-NR1R2-S-, avec R4 étant un groupe alkyle en $C_1$-$C_8$, saturé ou insaturé, et pouvant comprendre des liaisons amide, ester, éther et,
W étant soit un antagoniste des récepteurs δ, soit un antagoniste des récepteurs κ, ainsi que l'un de ses sels pharmaceutiquement acceptables.

2. Composé selon la revendication 1, **caractérisé en ce que**, si W est un antagoniste des récepteurs δ, il est choisi parmi le groupe consistant en le naltrindole, le Naltriben, le 7-benzylidenenaltrexone (BNTX), et le 7-(5', 6'-benzo-2'-spiro-indanyl)naltrexone (BSINTX), et si W est un antagoniste des récepteurs κ, il est choisi parmi le groupe consistant en le 5'-guanidinonaltrindole et le nor-binaltorphimine (nor-BNI).

3. Composé selon la revendication 1 ou 2, **caractérisé en ce que** R1 est un alkyle substitué et le substituant est choisi parmi le groupe consistant en un groupe alkyle en $C_1$-$C_5$ saturé ou insaturé ; un groupe amino ; un groupe COOR5, un groupe CONR5R6, R5 et R6 représentant indépendamment l'hydrogène, un groupe alkyle en $C_1$-$C_{20}$, saturé ou insaturé, un aryle, un hétéroaryle ; un alkyle en en $C_1$-$C_{20}$, de préférence en $C_1$-$C_{10}$, portant une fonction aldéhyde et/ou cétone.

4. Composé selon l'une quelconque des revendications 1 à 3, **caractérisé en ce que** R2 est substitué, et le substituant peut être un alkyle en $C_1$-$C_4$ saturé ou insaturé.

5. Composé selon l'une quelconque des revendications 1 à 4, **caractérisé en ce que** R4 représente un groupe $-(CH_2)_n-C(O)-NH-$ ou $-(CH_2)_n-NH-C(O)-$, dans lequel n est un entier compris entre 1 et 8, de préférence entre 1 et 4.

**6.** Composé selon l'une quelconque des revendications 1 à 5, **caractérisé en ce que** le composé présente une ou plusieurs des caractéristiques suivantes :

- R2 est un hydrogène ; et/ou
- R1 est un groupe alkyle linéaire $-(CH_2)_2-$ ; et/ou
- R4 est un groupe $-(CH_2)_2-C(O)-NH-$ ; et/ou
- W représente le naltrindole.

**7.** Composé selon l'une quelconque des revendications 1 à 6, **caractérisé en que** X est un radical -S-R4-W ou un radical $M_{R3}$6G-NR1R2-S-, les radicaux R1, R2, et R3 sur les deux radicaux $M_{R3}$6G-NR1R2-S- peuvent être identiques ou différents.

**8.** Composé selon la revendication 7, **caractérisé en que** le composé présente une formule sélectionnée parmi le groupe consistant en les formules (II) et (III) :

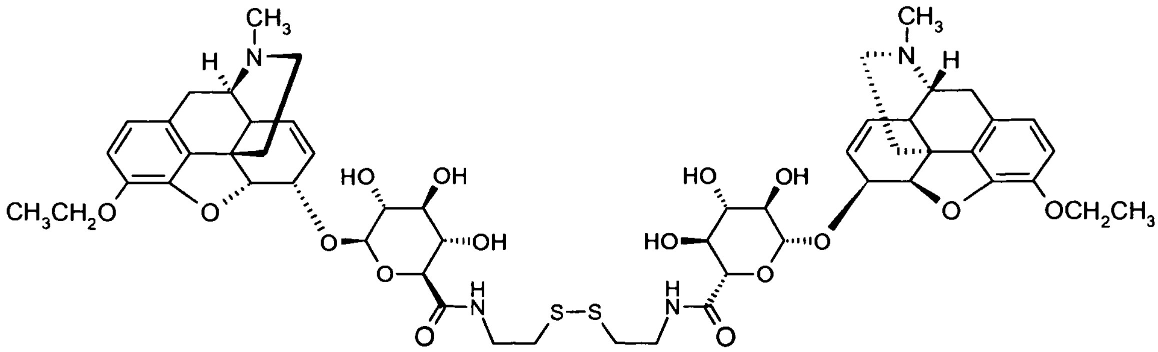

(II) : M3Et-6G-cya-cya-M3Et-6G

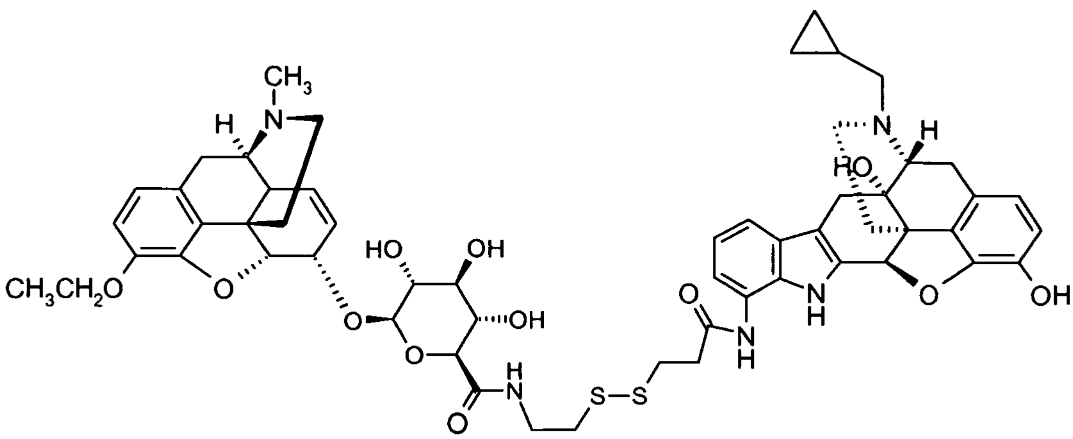

(III) : M3Et-6G-cya-3MP-NTI.

**9.** Composé selon l'une quelconque des revendications 1 à 8 en tant que médicament.

10. Composition pharmaceutique comprenant un composé selon l'une quelconque des revendications 1 à 8.

11. Composé selon l'une quelconque des revendications 1 à 8 pour le traitement de la douleur.

12. Composé selon l'une quelconque des revendications 1 à 8 pour le traitement de dysfonctions sexuelles.

**Claims**

1. A compound of formula (A) :

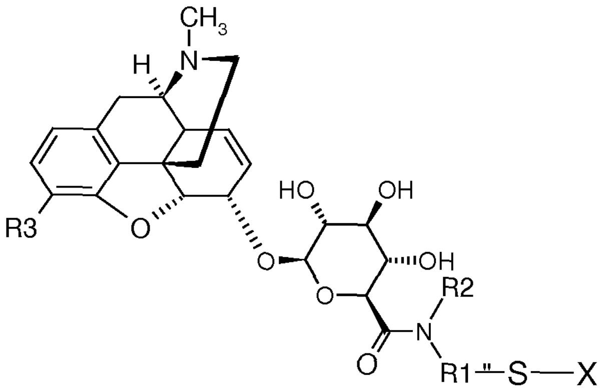

(A)

wherein,
the whole entity (A), except for substituent X, is designated as $M_{R3}6G-NR_1R_2-S$ ;
R1 is a saturated or unsaturated $C_1-C_{10}$ alkyl group, linear or branched, the alkyl chain being possibly interrupted by one or several heteroatom(s) chosen among O, S and N;
R2 is a hydrogen, a saturated or unsaturated $C_1-C_5$ alkyl group, linear or branched, or an aryl, heteroaryl or a ($C_1-C_5$) alkylaryl group;
R3 is a O-ethyl group;
X is a hydrogen, a radical -S-R4-W, or a radical $M_{R3}6G-NR_1R_2-S$,
with R4 being a $C_1-C_8$ alkyl group, saturated or unsaturated, that may include amide, ester, ether bonds and
W being either a $\delta$ receptor antagonist or a $\kappa$ receptor antagonist,
as well as one of its pharmaceutically acceptable salts.

2. The compound according to claim 1, wherein, if W is a $\delta$ receptor antagonist, it is chosen among the group consisting of naltrindole, naltriben, 7-benzylidenenaltrexone (BNTX) and 7-(5', 6'-benzo-2'-spiro-indanyl)naltrexone (BSINTX), and if W is a $\kappa$ receptor antagonist, it is chosen among the group consisting of 5'-guanidinonaltrindole and nor-binaltorphimine (nor-BNI).

3. The compound according to claim 1 or 2, wherein R1 is a substituted alkyl and the substituent is chosen among the group consisting of a saturated or unsaturated $C_1-C_5$ alkyl group; an amino group; a COOR5 group, a CONR5R6 group, R5 and R6 being independently hydrogen, a saturated or unsaturated $C_1-C_{20}$ alkyl group, an aryl, a heteroaryl; a $C_1-C_{20}$ alkyl, preferably in $C_1-C_{10}$, carrying an aldehyde and/or ketone function.

4. The compound according to any one of claims 1 to 3, wherein R2 is substituted and the substituent can be a saturated or unsaturated $C_1-C_4$ alkyl .

5. The compound according to any one of claims 1 to 4, wherein R4 is a - $(CH_2)_n$-C(O)-NH- group or -$(CH_2)_n$-NH- C (O)-, in which n is an integer comprised between 1 and 8, preferably between 1 and 4.

6. The compound according to any one of claims 1 to 5, wherein the compound shows one or several following characteristics :

- R2 is hydrogen ; and/or

- R1 is a linear alkyl $-(CH_2)_2$-group; and/or
- R4 is $-(CH_2)_2$-C(O)-NH- group; and/or
- W is naltrindole.

**7.** The compound according to any one of claims 1 to 6, wherein X is a radical -S-R4-W or a radical $M_{R3}6G-NR_1R_2-S$, radicals R1, R2, and R3 on both radicals $M_{R3}6G-NR_1R_2-S$ being either identical or different.

**8.** The compound according to claim 7, wherein the compound presents a formula chosen among the group consisting of formulae (II) and (III) :

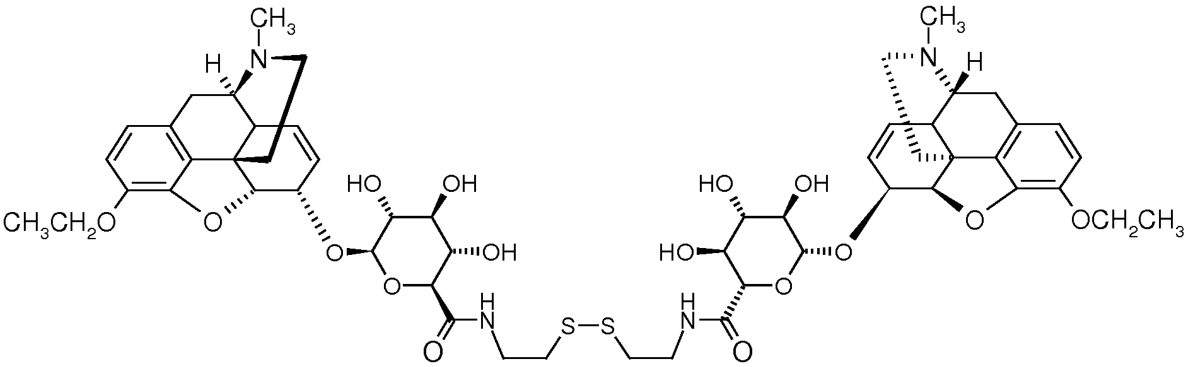

(II) : M3Et-6G-cya-cya-M3Et-6G

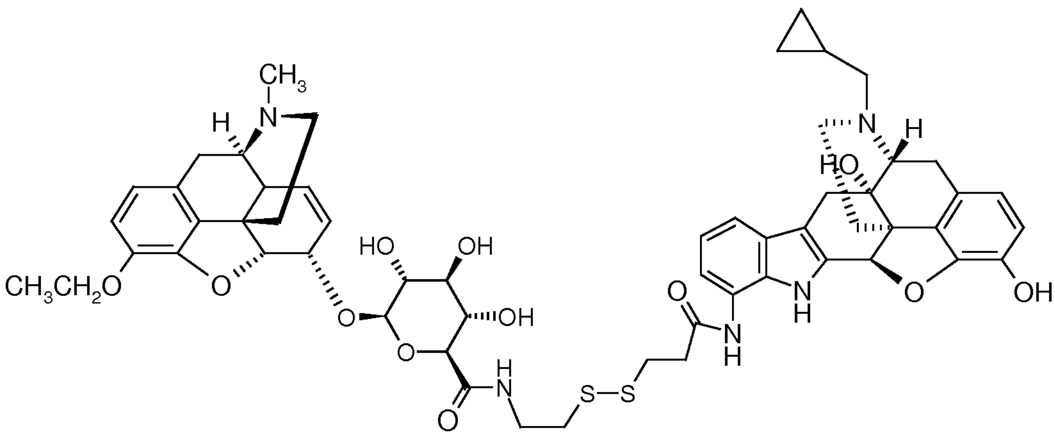

(III) : M3Et-6G-cya-3MP-NTI.

**9.** Compound according to any one of claims 1 to 8 as a drug.

**10.** A pharmaceutical composition comprising a compound according to any one of claims 1 to 8.

**11.** A compound according to any one of claims 1 to 8 for the treatment of pain.

**12.** A compound according to any one of claims 1 to 8 for the treatment of sexual dysfunctions.

**Patentansprüche**

**1.** Verbindung der Formel (A):

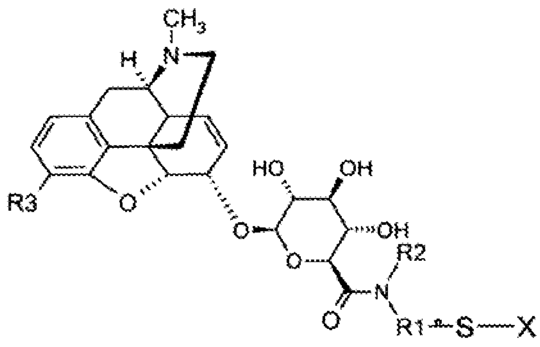

(A)

in der
die gesamte Einheit (A), ausgenommen Substituent X, als $M_{R3}$6G-NR1R2-S- bezeichnet wird; R1 für eine gesättigte oder ungesättigte, gerad- oder verzweigtkettige ($C_1$-$C_{10}$)-Alkylgruppe steht, wobei die Alkylkette gegebenenfalls von einem oder mehreren Heteroatomen unterbrochen ist, die aus O, S und N ausgewählt sind;
R2 für einen Wasserstoff, eine gesättigte oder ungesättigte, gerad- oder verzweigtkettige ($C_1$-$C_5$)-Alkylgruppe oder eine Aryl-, Heteroaryl- oder ($C_1$-$C_5$)-Alkylarylgruppe steht;
R3 für eine -O-Ethylgruppe steht;
X für einen Wasserstoff, einen -S-R4-W-Rest oder einen $M_{R3}$6G-NR1R2-S-Rest steht,
wobei R4 eine gesättigte oder ungesättigte ($C_1$-$C_8$)-Alkylgruppe ist und Amid-, Ester-, Etherbindungen umfassen kann, und
wobei W entweder ein δ-Rezeptor-Antagonist oder ein κ-Rezeptor-Antagonist ist,
sowie eines ihrer pharmazeutisch verträglichen Salze.

2. Verbindung gemäß Anspruch 1, **dadurch gekennzeichnet, dass**, falls W ein 8-Rezeptor-Antagonist ist, es aus der Gruppe ausgewählt ist, bestehend aus Naltrindol, Naltriben, 7-Benzylidennaltrexon (BNTX) und 7-(5', 6'-Benzo-2'-spiro-indanyl)naltrexon (BSINTX), und, falls W ein κ-Rezeptor-Antagonist ist, es aus der Gruppe ausgewählt ist, bestehend aus 5'-Guanidinnaltrindol und nor-Binaltorphimin (nor-BNI).

3. Verbindung gemäß Anspruch 1 oder 2, **dadurch gekennzeichnet, dass** R1 ein substituiertes Alkyl ist und der Substituent aus der Gruppe ausgewählt ist, bestehend aus einer gesättigten oder ungesättigten ($C_1$-$C_5$)-Alkylgruppe; einer Aminogruppe; einer COOR5-Gruppe; einer CONR5R6-Gruppe, wobei R5 und R6 unabhängig voneinander für Wasserstoff, eine gesättigte ($C_1$-$C_{20}$)-Alkylgruppe, ein Aryl, ein Heteroaryl; ein ($C_1$-$C_{20}$)-Alkyl, vorzugsweise $C_1$-$C_{10}$, das eine Aldehyd- und/oder Ketofunktion trägt, stehen.

4. Verbindung gemäß einem der Ansprüche 1 bis 3, **dadurch gekennzeichnet, dass** R2 substituiert ist und der Substituent ein gesättigtes oder ungesättigtes ($C_1$-$C_4$)-Alkyl sein kann.

5. Verbindung gemäß einem der Ansprüche 1 bis 4, **dadurch gekennzeichnet, dass** R4 für eine $-(CH_2)_n$-C(O)-NH-Gruppe oder $-(CH_2)_n$-NH-C(O)-Gruppe steht, worin n ein ganze Zahl zwischen 1 und 8, vorzugsweise zwischen 1 und 4, ist.

6. Verbindung gemäß einem der Ansprüche 1 bis 5, **dadurch gekennzeichnet, dass** die Verbindung ein oder mehrere der folgenden Charakteristika aufweist:

- R2 ist ein Wasserstoff; und/oder
- R1 ist eine geradkettige $-(CH_2)_2$-Alkylgruppe; und/oder
- R4 ist eine-$(CH_2)_2$-C(O)-NH-Gruppe; und/oder
- W steht für Naltrindol.

7. Verbindung gemäß einem der Ansprüche 1 bis 6, **dadurch gekennzeichnet, dass** X ein -S-R4-W-Rest oder ein $M_{R3}$6G-NR1R2-S-Rest ist und die Reste R1, R2 und R3 an den beiden $M_{R3}$6G-NR1R2-S-Resten identisch oder verschieden sein können.

**8.** Verbindung gemäß Anspruch 7, **dadurch gekennzeichnet, dass** die Verbindung eine Formel aufweist, die aus der Gruppe ausgewählt ist, bestehend aus den Formeln (II) und (III):

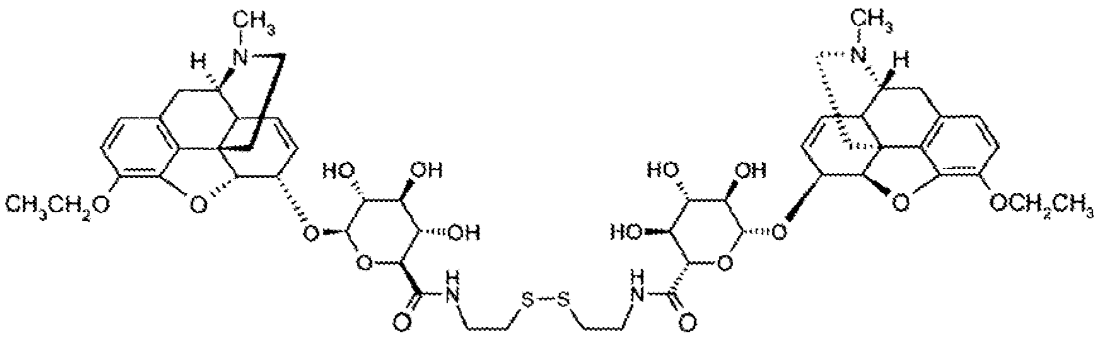

(II): M3Et-6G-cya-cya-M3Et-6G

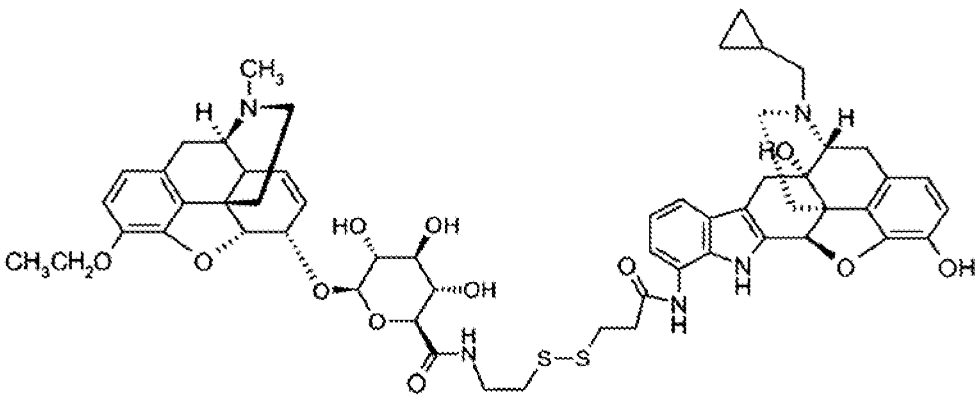

(III): M3Et-6G-cya-3MP-NTI.

**9.** Verbindung gemäß einem der Ansprüche 1 bis 8 als Medikament.

**10.** Pharmazeutische Zusammensetzung, umfassend eine Verbindung gemäß einem der Ansprüche 1 bis 8.

**11.** Verbindung gemäß einem der Ansprüche 1 bis 8 zur Schmerzbehandlung.

**12.** Verbindung gemäß einem der Ansprüche 1 bis 8 zur Behandlung von sexuellen Dysfunktionen.

# Figure 1

## Synthèse de C6G-Cya

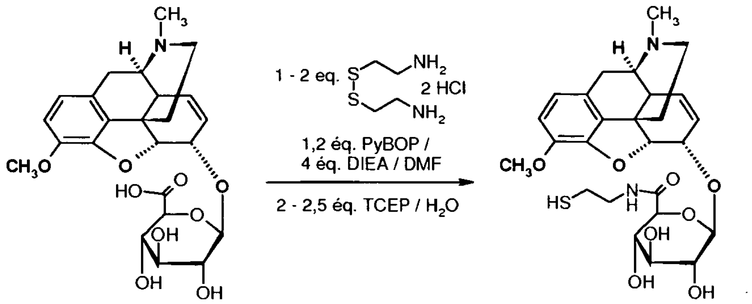


## Synthèse de M3Et-6G-Cya

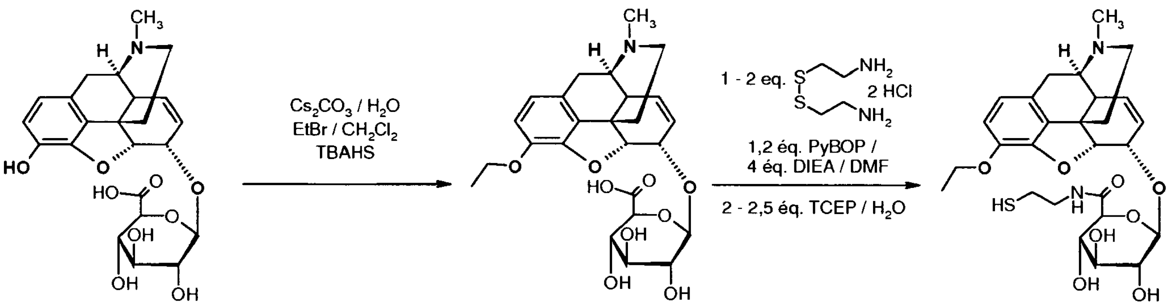


## Synthèse de M3Et-6G-Cya-cya-M3Et-6G

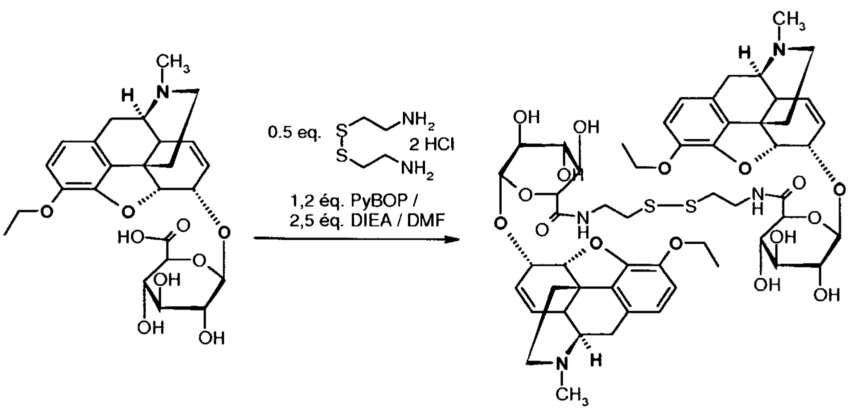

**Figure 1 (suite)**

**Synthèse de M3iPro-6G-cya-cya-M3iPro-6G**

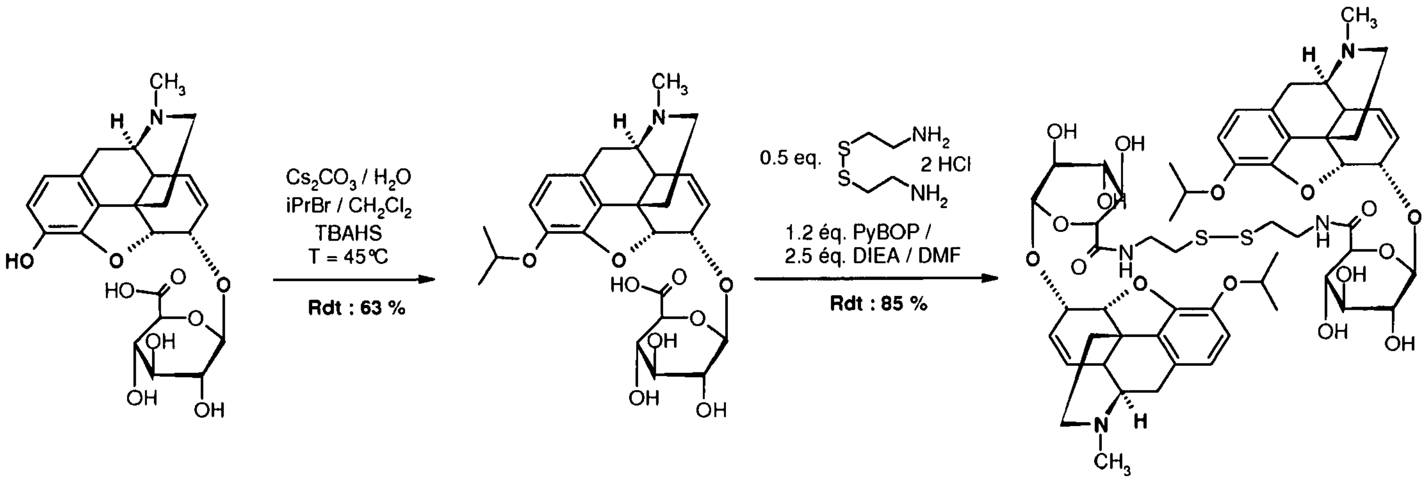

# Figure 1 (suite)

## Synthèse de M3Et-6G-cya-3MP-NTI

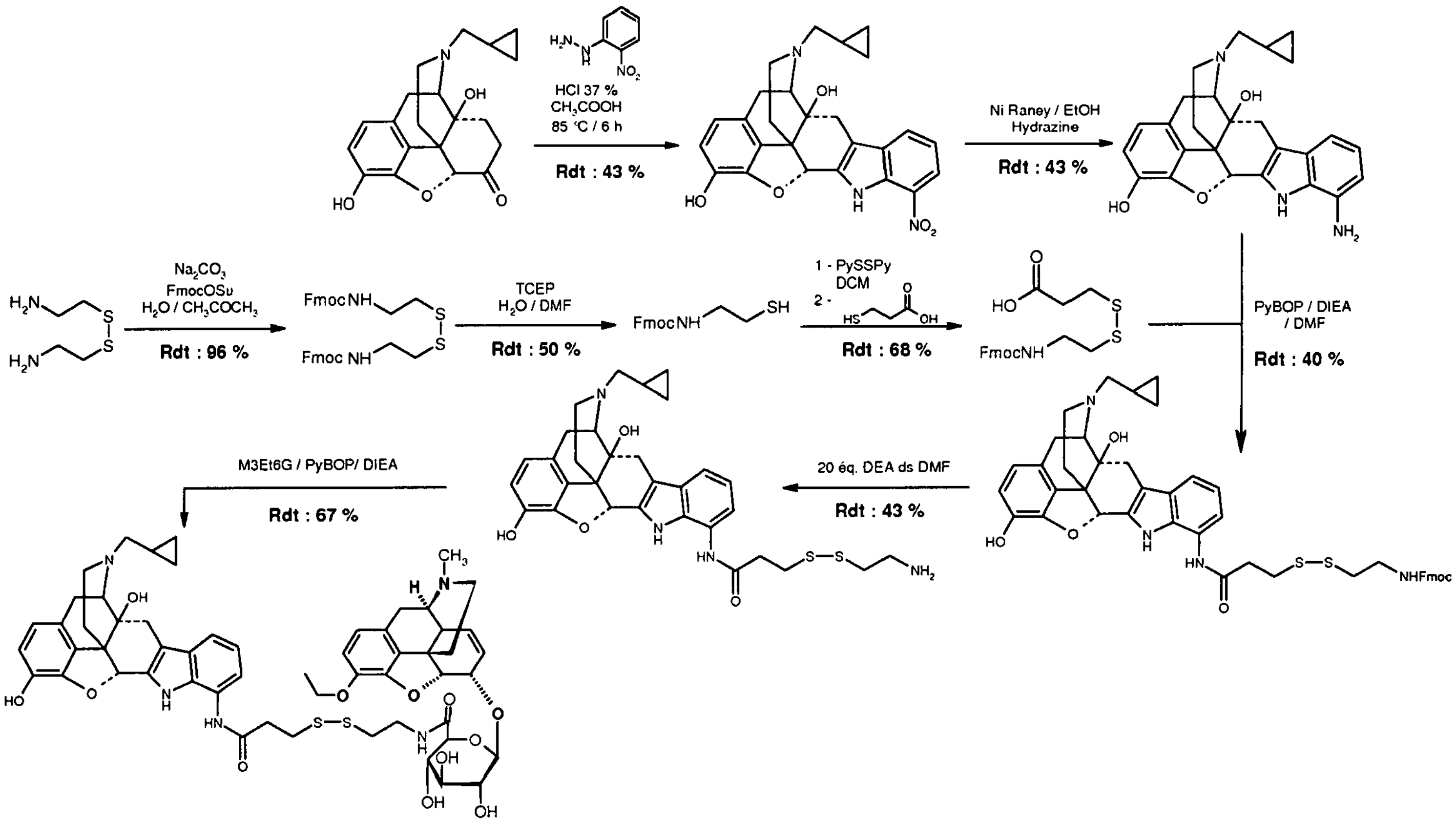

**Figure 2: Activité analgésique des composés dans le test du Tail Flick**

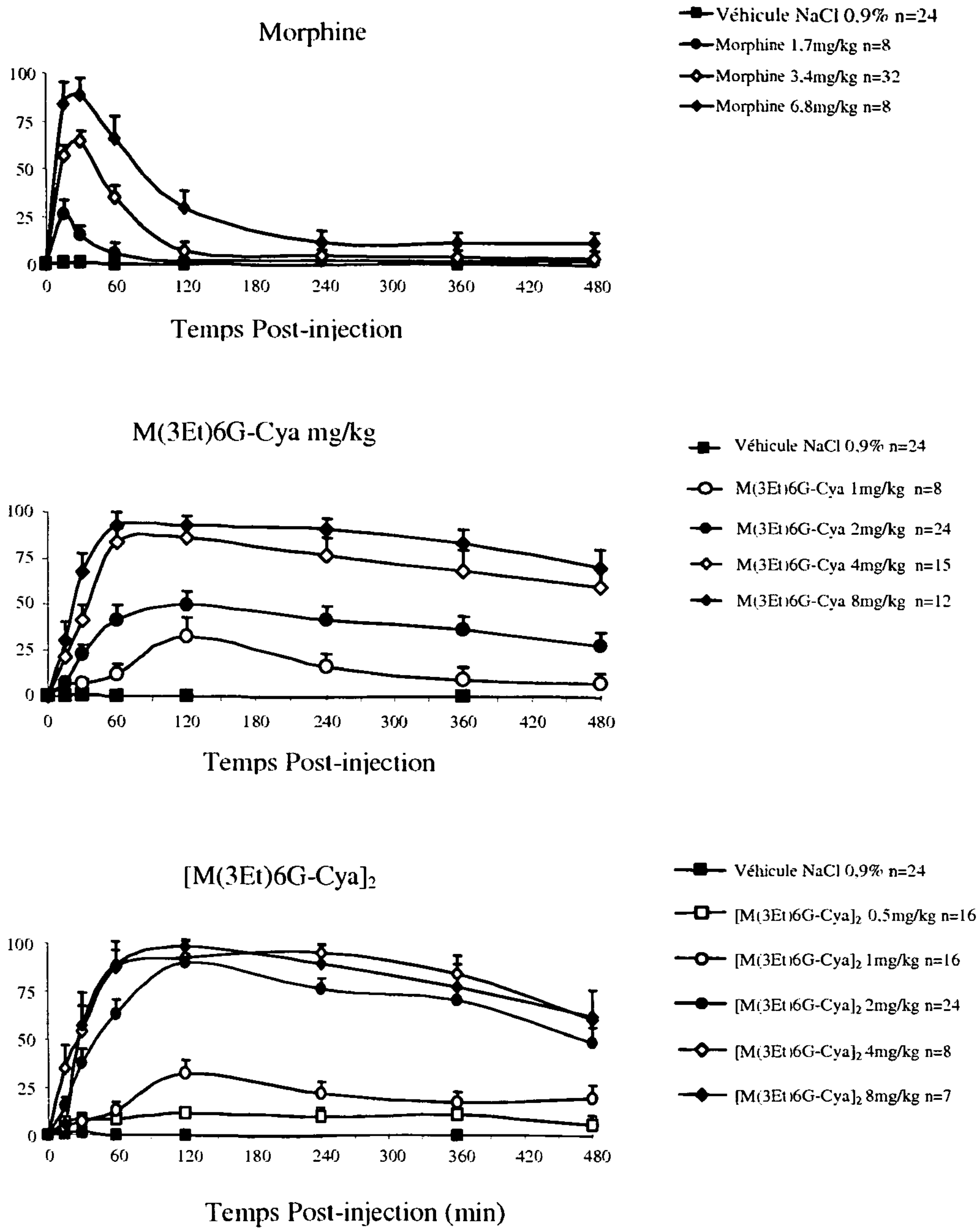

**Figure 3: Activité analgésique des composés dans le test de la Hot Plate**

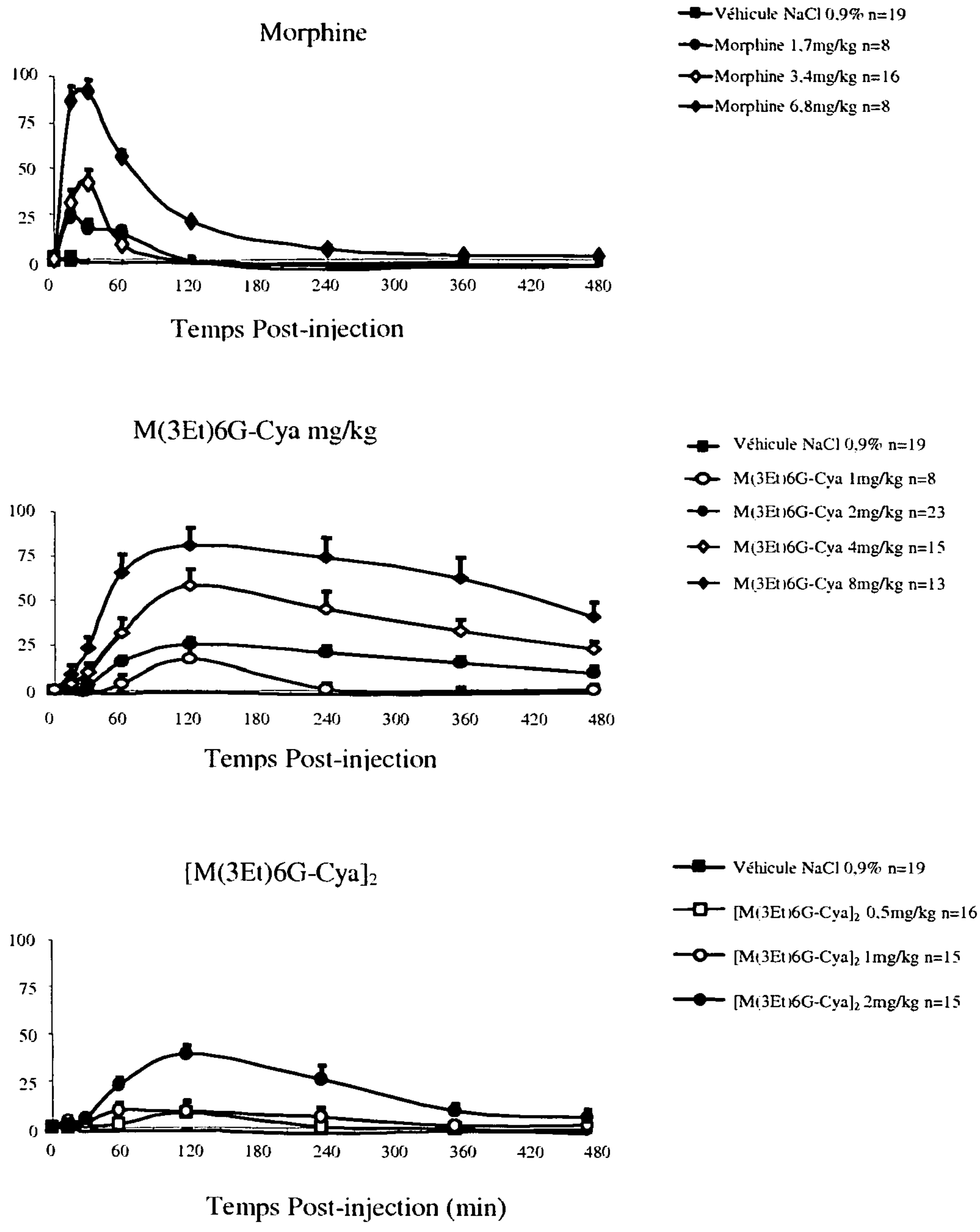

# Figure 4 : Influence des composés sur le transit intestinal

## Schéma du protocole :

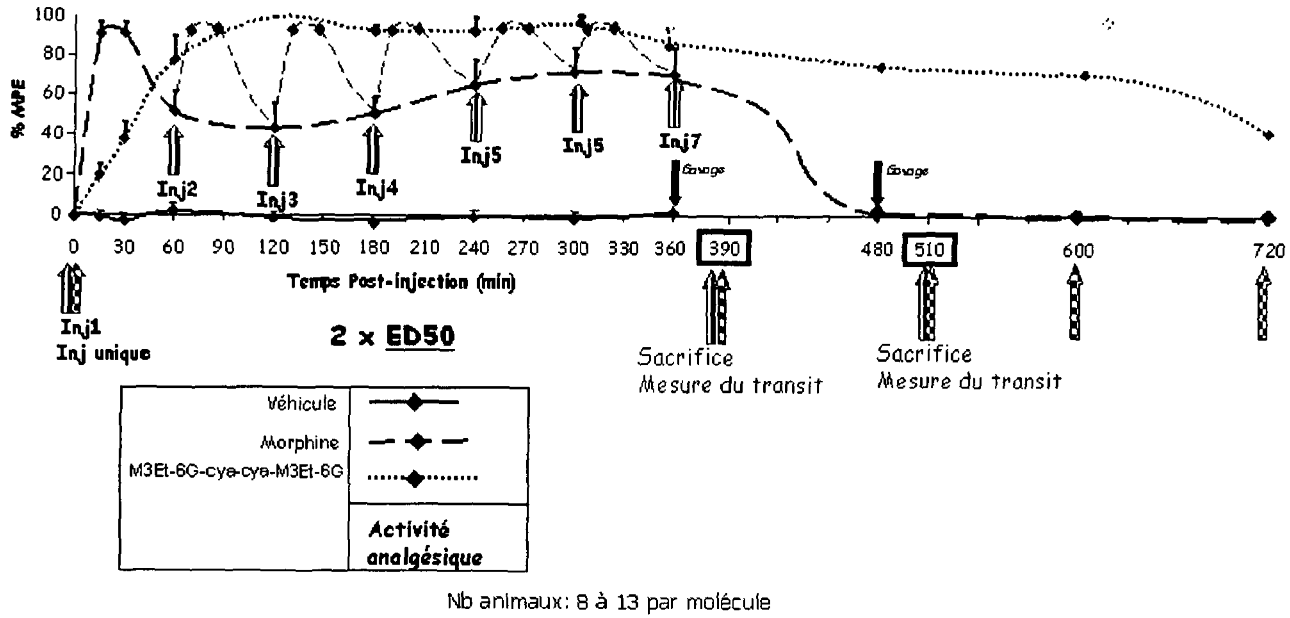

# Figure 4 (suite)

**Résultats** :

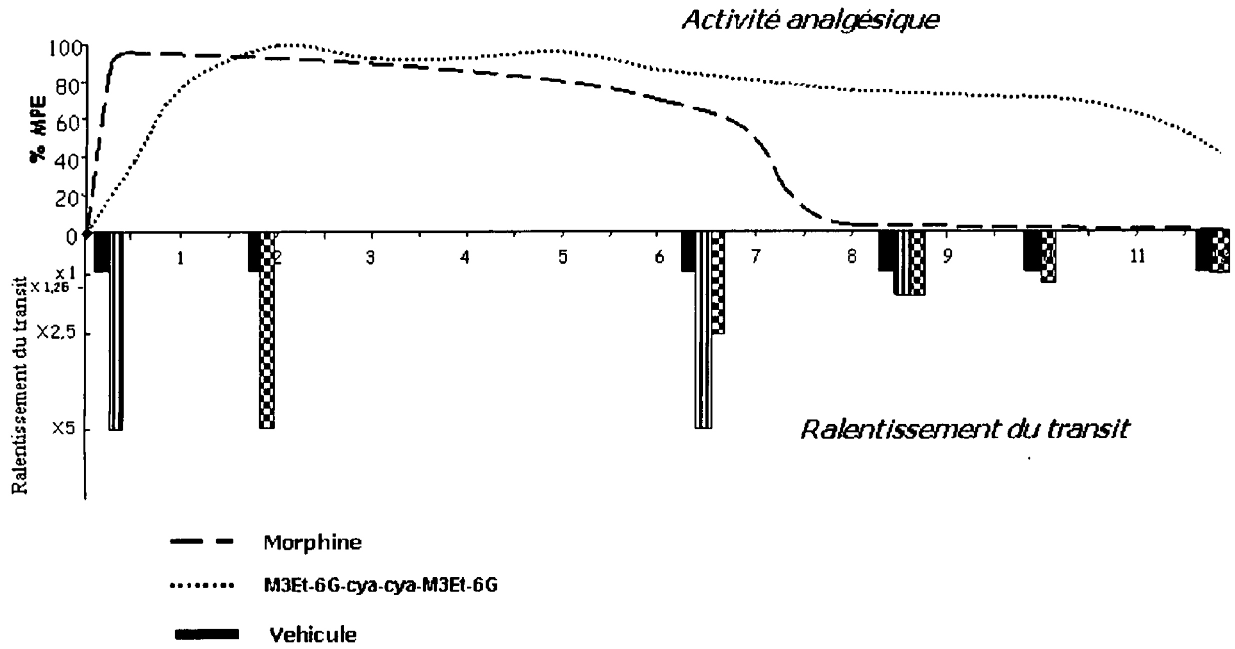

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 2005063263 A
- WO 2006073396 A
- EP 597915 B1
- US 6046313 A
- EP 1086114 B1
- WO 9505831 A
- EP 975648 B1
- EP 816375 A
- US 23186872 A
- US 06974839 B


### Littérature non-brevet citée dans la description


- **Minoru Nariata.** *Pharmacol et Ther,* 2001, vol. 89, 1-15
- **George SR.** *J Biol Chem,* 2000, vol. 275, 26128-26135
- **Jordan BA.** *Nature,* 1999, vol. 399, 697-700
- **Gomes I.** *J Neurosci,* 2000, vol. 20, RC110
- **Hazum.** *Biochem Biophys Res Comm,* 1982, vol. 104, 347-353
- **Rios.** *Pharm & Ther,* 2001, vol. 92, 71-87
- **Pasternak ; Wood.** *Life Sci,* 1986, vol. 38, 1889-1898
- **Rothman.** *J Subst Abuse Treat,* 2000, vol. 19, 277-281
- **Shook.** *Am Rev Respir Dis,* 1990, vol. 142, 895-909
- *J Neurosci,* 23 Mars 2005, vol. 25 (12), 3229-33
- *J Pharmacol Exp Ther.,* Mai 2001, vol. 297 (2), 597-605
- *J Pharmacol Exp Ther.,* Décembre 1998, vol. 287 (3), 815-23
- **Stein.** *Ann Med,* 1995, vol. 27 (2), 219-21
- **Janso, Stein.** *Curr Opin Pharmacol,* 2001, vol. 1 (1), 62-65
- **C. Stein.** *J of Pain and Symptom Management,* 1990, vol. 6 (3), 119-124
- **Zajaczowska R.** *Reg Anesth Pain Med,* 2004, vol. 29 (5), 424-429
- **Likar.** *Pain,* 1998, vol. 76 (1-2), 145-50
- **Tokuyama.** *Life Sci,* 1998, vol. 62 (17-18), 1677-81
- **Junien.** *Aliment Pharmacol Ther,* 1995, vol. 9 (2), 117-26
- **Dehave-Hudkins.** *J Pharmacol Exp The,* 1999, vol. 289 (1), 494-502
- **Stein.** *N Engl J Med,* 1991, vol. 325, 1123-1126
- **Daniels.** *PNAS,* 2005, vol. 102 (52), 19208
- **Paul.** *J Pharmacol. Exp. Ther.,* 1989, vol. 251, 477-483
- **Frances et al.** *J Pharmacol. Exp. Ther.,* 1992, vol. 262, 25-31
- *Current Topics in Medicinal Chemistry,* 2005, vol. 5, 585-594
- **Hidetoshi.** *Biochem Pharm.,* 1969, vol. 8 (2), 279-286
- **Frances.** *J Pharmacol. Exp. Ther.,* 1992, vol. 262, 25-31
- **G. Saito.** *Advanced Drug Delivery Reviews,* 2003, vol. 55, 199-215
- **Abdulghani A Houdi et al.** *Pharm Biochem and Beh,* 1996, vol. 53 (3), 665-671
- **Salvatella et al.** *Biiorg & Med Chem Lett.,* 2004, vol. 14, 905-908
- **Eledjam.** *Cah Anesthesiol.,* 1991, vol. 39 (2), 111-4
- **Safarinejad.** *J Clin Psychopharmacol.,* 2006, vol. 26 (1), 27-31
- **D'Amour ; Smith.** *Pharmacol Exp Ther,* 1941, vol. 72, 74-79